(19) Europäisches Patentamt
European Patent Office
Office européen des brevets

(11) Veröffentlichungsnummer: **0 547 445 A1**

(12) # EUROPÄISCHE PATENTANMELDUNG

(21) Anmeldenummer: **92120660.3**

(22) Anmeldetag: **03.12.92**

(30) Priorität: **16.12.91 DE 4141399**

(43) Veröffentlichungstag der Anmeldung:
**23.06.93 Patentblatt 93/25**

(84) Benannte Vertragsstaaten:
**BE CH DE ES FR GB IT LI NL**

(51) Int. Cl.$^5$: **C07D 207/38**, A01N 43/36

(71) Anmelder: **BAYER AG**

**W-5090 Leverkusen 1 Bayerwerk(DE)**

(72) Erfinder: **Baasner, Bernd, Dr.**
**Wagnerstrasse 83**
**W-5060 Bergisch Gladbach 2(DE)**
Erfinder: **Fischer, Reiner, Dr.**
**Nelly-Sachs-Strasse 23**
**W-4019 Monheim(DE)**
Erfinder: **Widdig, Arno, Dr.**
**Eifgenstrasse 8**
**W-5068 Odenthal-Blecher(DE)**
Erfinder: **Lürssen, Klaus, Dr.**
**August-Kierspel-Strasse 145**
**W-5060 Bergisch Gladbach 2(DE)**
Erfinder: **Santel, Hans-Joachim, Dr.**
**Grünstrasse 9a**
**W-5090 Leverkusen 1(DE)**
Erfinder: **Schmidt, Robert R., Dr.**
**Im Waldwinkel 110**
**W-5060 Bergisch Gladbach 2(DE)**

(54) **4-(Substituierte)Amino-3-arylpyrrolinon-2-Derivate und ihre Verwendung als Herbizide.**

(57) Die Erfindung betrifft neue 4-(substituierte)Amino-3-arylpyrrolinon-Derivate der allgemeinen Formel (I)

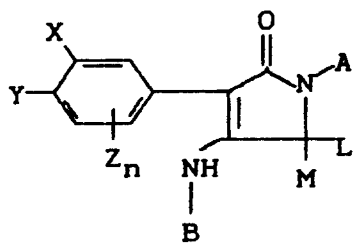

(I)

in welcher
X, Y, Z, n, B, M, L und A die in der Beschreibung genannten Definitionen haben, ein Verfahren, sowie neue Zwischenprodukte zu ihrer Herstellung und ihre Verwendung als Herbizide.

EP 0 547 445 A1

Die Erfindung betrifft neue 4-(substituierte)Amino-3-arylpyrrolinon-Derivate, Verfahren sowie neue Zwischenprodukte zu ihrer Herstellung und ihre Verwendung als Herbizide.

Von 3-Acyl-pyrrolidin-2,4-dionen sind pharmazeutische Eigenschaften vorbeschrieben (S. Suzuki et. al., Chem. Pharm. Bull. 15 1120 (1967)). Weiterhin wurden N-Phenylpyrrolidin-2,4-dione von R. Schmierer und H. Mildenberger, Liebigs Ann. Chem. 1985 1095, synthetisiert. Eine biologische Wirksamkeit dieser Verbindungen wurde nicht beschrieben.

In EP-A 0 262 399 werden ähnlich strukturierte Verbindungen (3-Aryl-pyrrolidin-2,4-dione) offenbart, von denen jedoch keine herbizide Wirksamkeit bekannt geworden ist.

In EP-A-0 415 185 werden 4-Alkoxy bzw. 4-substituierte Amino-3-arylpyrrolinon-Derivate und ihre herbizide Verwendung offenbart.

Weiterhin werden in der EP-A 377 893 3-Arylpyrrolidin-2,4-dion-Derivate mit insektizider, akarizider und herbizider Wirkung beschrieben, in denen in 4-Stellung des Pyrrolinons Hydroxy stehen kann. In der o.g. Anmeldung EP-A-415 185 wörtlich genannte Verbindungen werden mittels Disclaimers vom Schutzumfang der vorliegenden Anmeldung ausgeschlossen.

Es wurden nun neue 4-(substituierte) Amino-3-arylpyrrolinon-Derivate der allgemeinen Formel (I) gefunden

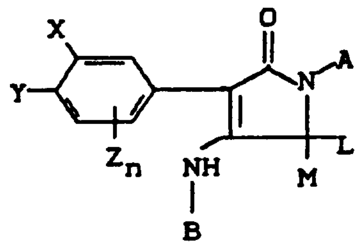

(I)

in welcher

X und Y unabhängig voneinander für Wasserstoff, Halogen, Alkyl, Alkoxy, Alkylthio, Halogenalkoxy, Halogenalkyl, oder jeweils für einen unsubstituierten oder substituierten Rest der Reihe Aryl, Aryloxy oder Arylthio stehen,

Z für Halogen, Alkyl oder Alkoxy steht,

n für eine Zahl O, 1, 2 oder 3 steht,

A für einen jeweils unsubstituierten oder jeweils durch Halogen substituierten Rest der Reihe Alkyl, Alkenyl, Alkinyl, Alkoxyalkyl, Halogenalkyl, Polyalkoxyalkyl oder Alkylthioalkyl, gegebenenfalls durch Heteroatome unterbrochenes Cycloalkyl, oder für unsubstituiertes oder durch Halogen, Alkyl, Halogenalkyl, Alkoxy und/oder Nitro substituiertes Arylalkyl oder unsubstituiertes oder substituiertes Aryl steht, wobei als Substituenten Halogen, Alkyl, Alkoxy, Halogenalkyl, Halogenalkoxy, jeweils unsubstituiertes oder durch Halogen, Alkyl, Alkoxy, Halogenalkyl und/oder Halogenalkoxy substituiertes Phenoxy oder Phenylthio in Frage kommen,

B für Wasserstoff, für die Gruppe

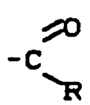

oder für die Gruppe

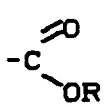

steht,

L für Wasserstoff, oder für einen gegebenenfalls durch Halogen substituierten Rest der Reihe Alkyl, Alkenyl, Alkinyl, Polyalkoxyalkyl, Alkoxyalkyl, Alkylthioalkyl, gegebenenfalls durch Heteroatome unterbrochenes Cycloalkyl oder für einen gegebenenfalls durch Halogen,

Alkyl, Halogenalkyl, Alkoxy oder Nitro substituierten Rest der Reihe Aryl, Arylalkyl oder Hetaryl steht,

M für Wasserstoff, Alkyl oder Alkoxyalkyl steht,

oder

A und L oder L und M gemeinsam mit den Atomen an die sie gebunden sind einen Cyclus bilden und

R für einen gegebenenfalls durch Halogen substituierten Rest der Reihe Alkyl, Alkenyl, Alkinyl, Alkoxyalkyl, Polyalkoxyalkyl, Alkylthioalkyl, gegebenenfalls durch Heteroatome unterbrochenes Cycloalkyl oder für ein gegebenenfalls durch Halogen, Alkyl, Halogenalkyl, Alkoxy oder Nitro substituiertes Aryl steht,

sowie die enantiomeren Formen von Verbindungen der Formel (I), ausgenommen die Verbindungen 4-Amino-1-isopropyl-3-(3-trifluormethylphenyl)-3-pyrrolin-2-on und 4-Amino-1-cyclopropyl-3-(3-trifluormethylphenyl)-3-pyrrolin-2-on (EP 0 415 185).

Des weiteren seien die folgenden Untergruppen definiert;

(Ia): Verbindungen der Formel (I) worin B = Wasserstoff

(Ib): Verbindungen der Formel (I) worin B = -COR

(Ic): Verbindungen der Formel (I) worin B = -COOR.

Die aliphatischen Kohlenstoffketten, wie beispielsweise Alkyl, Halogenalkyl, Arylalkyl oder Phenoxyalkyl sind jeweils geradkettig oder verzweigt.

Bei substituierten Systemen, wie beispielsweise substituiertes Alkyl, Alkenyl, Cycloalkyl oder Aryl, kann die Substitution jeweils einfach oder mehrfach, gleich oder verschieden erfolgen. Aromatische Systeme werden bevorzugt einfach bis fünffach, insbesondere einfach bis dreifach, cyclische Systeme bevorzugt einfach bis achtfach, insbesondere einfach bis fünffach, substituiert.

Weiterhin wurde gefunden, daß man die Verbindungen der Formel (Ia),

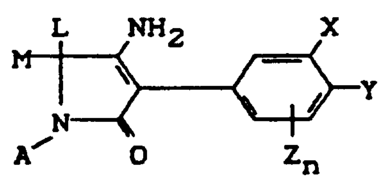

(Ia)

in denen

aα) B für Wasserstoff steht und in welcher die Reste A, L, M, X, Y und $Z_n$ die oben angegebenen Bedeutungen haben,

erhält, wenn man 4-Hydroxypyrrolinone der Formel (II)

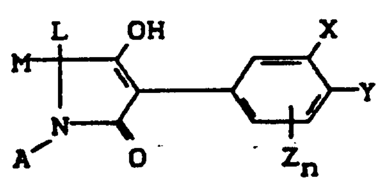

(II)

in denen A, L, M, X, Y, Z und n die oben angegebenen Bedeutungen haben,

mit Ammoniak oder Ammoniaksalzen, gegebenenfalls in Gegenwart eines Verdünnungsmittels, gegebenenfalls in Gegenwart eines Katalysators und gegebenenfalls in Gegenwart eines wasserentziehenden Mittels, umsetzt,

oder daß man

aβ) Verbindungen der Formel (III)

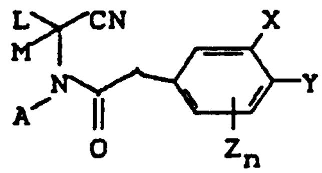

(III)

in denen
A, L, M, X, Y, Z und n die oben angegebenen Bedeutungen haben,
in Gegenwart eines Verdünnungsmittels und in Gegenwart einer Base intramolekular cyclisiert.

Außerdem wurde gefunden, daß man Verbindungen der Formel (Ib)

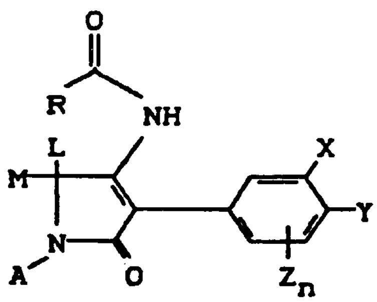

(Ib)

in denen
A, L, M, R, X, Y, Z und n die oben angegebenen Bedeutungen haben,
erhält, wenn man Verbindungen der Formel (Ia)

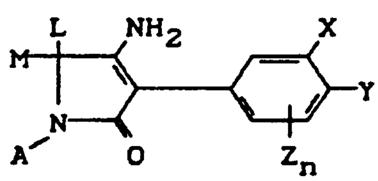

(Ia)

in denen
A, L, M, X, Y, Z und n die oben angegebenen Bedeutungen haben,
bα) mit Säurehalogeniden der allgemeinen Formel (IV)

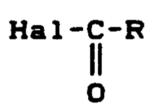

(IV)

in welcher

R die oben angegebene Bedeutung hat und

Hal für Halogen, insbesondere Chlor und Brom steht,

gegebenenfalls in Gegenwart eines Verdünnungsmittels und gegebenenfalls in Gegenwart eines Säurebindemittels umsetzt,
oder
bβ) mit Carbonsäureanhydriden der allgemeinen Formel (V)

R-CO-O-CO-R (V)

in welcher

R die oben angegebene Bedeutung hat,

gegebenenfalls in Gegenwart eines Verdünnungsmittels und gegebenenfalls in Gegenwart eines Säurebindemittels,

umsetzt.

(c) Ferner wurde gefunden, daß man Verbindungen der Formel (Ic)

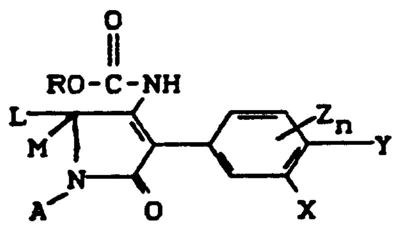

(Ic)

in welcher

A, L, M, X, Y, Z, R und n die oben angegebenen Bedeutungen haben,

erhält, wenn man Verbindungen der Formel (Ia)

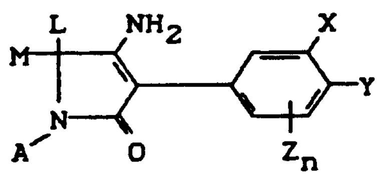

(Ia)

in welcher

A, L, M, X, Y, Z und n die oben angegebenen Bedeutungen haben,

mit Chlorameisensäureester der allgemeinen Formel (VI)

R-O-CO-Cl (VI)

in welcher

R die oben angegebene Bedeutung hat,

gegebenenfals in Gegenwart eines Verdünnungsmittels und gegebenenfalls in Gegenwart eines Saurebindemittels, umsetzt.

Schließlich wurde gefunden, daß die neuen 4-(substituierten)-Amino-3-arylpyrrolinone der allgemeinen Formel (I) herbizide Eigenschaften besitzen.

Überraschenderweise zeigen die erfindungsgemäßen 4-(substituierten)-Amino-3-arylpyrrolinone der allgemeinen Formel (I) eine hervorragende herbizide Wirksamkeit und gleichzeitig eine ausgezeichnete Verträglichkeit gegenüber wichtigen Kulturpflanzen.

Bevorzugt sind Verbindungen der Formel (I), bei welchen

X und Y unabhängig voneinander für Wasserstoff, Fluor, Chlor, Brom, Iod, $C_1$-$C_6$-Alkyl, $C_1$-$C_6$-Alkoxy, $C_1$-$C_6$-Alkylthio, $C_1$-$C_6$-Halogenalkoxy, Halogen-$C_1$-$C_4$-alkyl, oder jeweils für einen unsubstituierten oder einfach bis fünffach, gleich oder verschieden substituierten Rest der Reihe Phenyl, Phenoxy oder Phenylthio stehen, wobei als Phenylsubstituenten ausgewählt sind: Fluor, Chlor, Brom, Jod, $C_1$-$C_4$-Alkyl,$C_1$-$C_4$-Alkoxy und Halogen-$C_1$-$C_4$-alkyl,

Z für Fluor, Chlor, Brom, Iod, $C_1$-$C_6$-Alkyl oder $C_1$-$C_6$-Alkoxy steht,

n für eine Zahl 0, 1, 2 oder 3 steht,

A für einen jeweils unsubstituierten oder jeweils durch Halogen substituierten Rest der Reihe $C_1$-$C_{12}$-Alkyl, $C_3$-$C_8$-Alkenyl, $C_3$-$C_8$-Alkinyl, Halogen-$C_1$-$C_8$-alkyl, $C_1$-$C_{10}$-Alkoxy-$C_2$-$C_8$-alkyl, $C_1$-$C_8$-Polyalkoxy-$C_1$-$C_8$-alkyl, $C_1$-$C_{10}$-Alkylthio-$C_1$-$C_8$-alkyl oder für Cycloalkyl mit 3 bis 8 Ringatomen steht, das durch Sauerstoff, Stickstoff und/oder Schwefel unterbrochen sein kann, oder A für einen jeweils unsubstituierten oder im Phenylteil einfach bis fünffach, gleich oder verschieden substituierten Rest der Reihe Phenyl-$C_1$-$C_6$-alkyl, Phenyl oder Naphthyl steht, wobei als Substituenten jeweils ausgewählt sind: Fluor, Chlor, Brom, Iod, $C_1$-$C_6$-Alkyl,$C_1$-$C_6$-Alkoxy, Halogen-$C_1$-$C_6$-alkyl, Halogen-$C_1$-$C_6$-alkoxy oder jeweils unsubstituiertes

oder einfach bis fünffach, gleich oder verschieden durch Fluor, Chlor, Brom, Iod, $C_1$-$C_4$-Alkyl, $C_1$-$C_4$-Alkoxy, Halogen-$C_1$-$C_4$-alkyl und Halogen-$C_1$-$C_4$-alkoxy substituiertes Phenoxy oder Phenylthio,

B für Wasserstoff, für die Gruppe

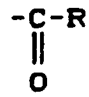

oder für die Gruppe

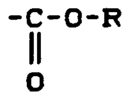

steht,

L für Wasserstoff, oder für einen gegebenenfalls durch Halogen substituierten geradkettigen oder verzweigten Rest der Reihe $C_1$-$C_{12}$-Alkyl, $C_3$-$C_8$-Alkenyl, $C_3$-$C_8$-Alkinyl, $C_1$-$C_{10}$-Alkoxy-$C_2$-$C_8$-alkyl, $C_1$-$C_8$-Polyalkoxy-$C_2$-$C_8$-alkyl, $C_1$-$C_{10}$-Alkylthio-$C_2$-$C_8$-alkyl, Cycloalkyl mit 3-8 Ringatomen, das durch Sauerstoff und/oder Schwefel unterbrochen sein kann, oder für einen gegebenenfalls durch Halogen, $C_1$-$C_6$-Alkyl-$C_1$-$C_6$-halogenalkyl, $C_1$-$C_6$-Alkoxy oder Nitro substituierten Rest der Reihe Aryl, Hetaryl oder Aryl-$C_1$-$C_6$-alkyl steht,

M für Wasserstoff oder für einen geradkettigen oder verzweigten Rest der Reihe $C_1$-$C_{12}$-Alkyl, $C_1$-$C_8$-Alkoxyalkyl steht,

oder worin

A und L oder L und M gemeinsam mit den Atomen, an die sie gebunden sind, einen 3 bis 8-gliedrigen Ring bilden, der gesättigt oder ungesättigt sein kann und der substituiert sein kann und/oder durch Sauerstoff/Schwefel unterbrochen sein kann,

R für einen gegebenenfalls durch Halogen substituierten geradkettigen oder verzweigten Rest der Reihe $C_1$-$C_{12}$-Alkyl, $C_3$-$C_8$-Alkenyl, $C_3$-$C_8$-Alkinyl, $C_1$-$C_{10}$-Alkoxy-$C_2$-$C_8$-alkyl, $C_1$-$C_8$-Polyalkoxy-$C_2$-$C_8$-alkyl, $C_1$-$C_{10}$-Alkylthio-$C_2$-$C_8$-alkyl, Cycloalkyl mit 3-8 Ringatomen, das durch Sauerstoff und/oder Schwefel unterbrochen sein kann, oder für gegebenenfalls durch Halogen, $C_1$-$C_6$-Alkyl-$C_1$-$C_6$-halogenalkyl-, $C_1$-$C_6$-Alkoxy oder Nitro substituiertes Aryl, steht,
sowie die enantiomeren Formen von Verbindungen der Formel (I) ausgenommen die Verbindungen: 4-Amino-1-cyclopropyl-3-(3-trifluormethylphenyl)-3-Pyrrolin-2-on und 4-Amino-1-isopropyl-3-(3-trifluormethylphenyl)-3-pyrrolin-2-on.

Besonders bevorzugt ist die Gruppe von Verbindungen der Formel (I), bei welchen

X für Wasserstoff, Fluor, Chlor, Brom, Methyl, Ethyl, n-oder iso-Propyl, Methoxy, Ethoxy, n- oder i-Propoxy, Halogenmethyl mit 1, 2 oder 3 Fluor- und/oder Chloratomen, Halogenethyl mit 1 bis 5, insbesondere 1 bis 3, Fluor- und/oder Chloratomen, $C_1$-$C_3$-Halogenalkoxy, $C_1$-$C_3$-Alkylthio, jeweils unsubstituiertes oder einfach bis fünffach, insbesondere einfach bis dreifach, gleich oder verschieden substituiertes Phenyl, Phenoxy oder Phenylthio steht, wobei als Phenylsubstituenten ausgewählt sind: Fluor, Chlor, Brom, Methyl, Ethyl, n- oder iso-Propyl, Methoxy, Ethoxy, n- oder iso-Propoxy und Trifluormethyl,

Y für Wasserstoff, Fluor, Chlor, Brom, Methyl, Ethyl, Methoxy, Ethoxy, Trifluormethoxy oder Trifluormethyl steht,

Z für Fluor oder Chlor steht,

n für 0, 1, 2 oder 3 steht und

A für Methyl, Ethyl, für einen jeweils geradkettigen oder verzweigten Rest der Reihe Propyl, Butyl, Pentyl, Hexyl, Heptyl, Octyl, Nonyl, Decyl, Halogen-$C_1$-$C_6$-alkyl, $C_3$-$C_6$-Alkenyl, $C_3$-$C_6$-Alkinyl, $C_1$-$C_4$-Alkoxy-$C_1$-$C_4$-alkyl, $C_1$-$C_4$-Alkylthio-$C_1$-$C_4$-alkyl steht, oder für Cycloalkyl mit 3 bis 6 Ringatomen, das durch Sauerstoff, Stickstoff oder Schwefel unterbrochen sein kann steht, oder A für einen jeweils unsubstituierten oder einfach bis fünffach, insbesondere einfach bis dreifach, gleich oder verschieden substituierten Rest der Reihe Phenyl, Benzyl oder Phenethyl steht, wobei als Substituenten jeweils ausgewählt sind: Fluor, Chlor, Methyl, Ethyl, n-oder iso-Propyl, Methoxy, Ethoxy, Trifluormethyl, Trifluormethoxy oder jeweils unsubstituiertes oder einfach bis dreifach,

gleich oder verschieden, durch Fluor, Chlor, Methyl, Methoxy, Trifluormethyl und Trifluormethoxy substituiertes Phenoxy oder Phenylthio,

B für Wasserstoff, für die Gruppe

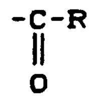

oder für die Gruppe

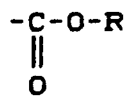

steht,

L für Wasserstoff, oder für einen gegebenenfalls durch Halogen substituierten geradkettigen oder verzweigten Rest der Reihe $C_1$-$C_{10}$-Alkyl, $C_3$-$C_6$-Alkenyl, $C_3$-$C_6$-Alkinyl, $C_1$-$C_8$-Alkoxy-$C_2$-$C_6$-alkyl, $C_1$-$C_6$-Polyalkoxy-$C_2$-$C_6$-alkyl, $C_1$-$C_8$-Alkylthio-$C_2$ $C_6$-alkyl, Cycloalkyl mit 3 bis 7 Ringatomen, das durch 1 bis 2 Sauerstoff- und/oder Schwefelatome unterbrochen sein kann oder gegebenenfalls durch Halogen, $C_1$-$C_4$-Alkyl, $C_1$-$C_4$-Halogenalkyl, $C_1$-$C_4$-Alkoxy oder Nitro substituiertes Aryl, steht,

M für Wasserstoff, oder für einen geradkettigen oder verzweigten Rest der Reihe $C_1$-$C_{10}$-Alkyl, $C_1$-$C_6$-Alkoxyalkyl steht,

oder worin

A und L oder L und M gemeinsam mit den Atomen an die sie gebunden sind, einen 3 bis 7-gliedrigen Ring bilden, der gesättigt oder ungesättigt sein kann, der durch $C_1$-$C_6$-Alkyl, $C_1$-$C_4$-Alkoxy, $C_1$-$C_2$-Halogenalkyl oder Phenyl sustituiert sein kann und/oder durch Sauerstoff/Schwefel unterbrochen sein kann,

R für einen gegebenenfalls durch Halogen substituierten geradkettigen oder verzweigten Rest der Reihe $C_1$-$C_{10}$-Alkyl, $C_3$-$C_6$-Alkenyl, $C_3$-$C_6$-Alkinyl, $C_1$-$C_8$-Alkoxy-$C_2$-$C_6$-alkyl, $C_1$-$C_6$-Polyalkoxy-$C_2$-$C_6$-alkyl, $C_1$-$C_8$-Alkylthio-$C_2$-$C_6$-alkyl, Cycloalkyl mit 3 bis 7 Ringatomen, das durch 1 bis 2 Sauerstoff- und/oder Schwefelatome unterbrochen sein kann oder für gegebenenfalls durch Halogen, $C_1$-$C_4$-Alkyl, $C_1$-$C_4$-Halogenalkyl, $C_1$-$C_4$-Alkoxy oder Nitro substituiertes Aryl, steht,

sowie die enantiomeren Verbindungen der Formel (I) ausgenommen die Verbindungen: 4-Amino-1-cyclopropyl-3-(3-trifluormethylphenyl)-3-pyrrolin-2-on und 4-Amino-1-isopropyl-3-(3-trifluormethylphenyl)-3-pyrrolin-2-on.

Ganz besonders bevorzugt ist die Gruppe von Verbindungen der Formel (I) bei welchen

X für Wasserstoff, Fluor, Chlor, Brom, Methyl, Ethyl, Methoxy, Ethoxy, n- oder i-Propoxy, Trifluormethyl, Trifluormethoxy oder für jeweils unsubstituiertes oder einfach bis dreifach, gleich oder verschieden substituiertes Phenyl oder Phenoxy steht, wobei als Substituenten ausgewählt sind: Fluor, Chlor, Methyl und Trifluormethyl,

Y für Wasserstoff, Fluor, Chlor, Brom, Methyl, Methoxy, Trifluormethoxy oder Trifluormethyl steht,

n für 0 steht und

A für Methyl, Ethyl, n-Propyl, iso-Propyl, n-, iso-, sec.- oder tert.-Butyl, für einen jeweils geradkettigen oder verzweigten Rest der Reihe Pentyl, Hexyl, Heptyl, Octyl, Halogen-$C_1$-$C_3$-alkyl, Allyl, Propargyl, Methoxymethyl, Methoxyethyl, Ethoxymethyl, Ethoxyethyl, Cyclopropyl, Cyclopentyl, Cyclohexyl, oder für jeweils unsubstituiertes oder einfach bis dreifach, gleich oder verschieden durch Fluor, Chlor, Trifluormethyl, Phenoxy und 4-Trifluormethyl-phenoxy substituiertes Phenyl oder Benzyl steht,

B für Wasserstoff, für die Gruppe -CO-R oder für die Gruppe

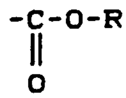

steht,

L für Wasserstoff, oder für einen gegebenenfalls durch Halogen substituierten geradkettigen oder verzweigten Rest der Reihe $C_1$-$C_8$-Alkyl, $C_3$-$C_4$-Alkenyl, $C_3$-$C_4$-Alkinyl, $C_1$-$C_6$-Alkoxy-$C_2$-$C_4$-alkyl, $C_1$-$C_4$-Polyalkoxy-$C_2$-$C_4$-alkyl, $C_1$-$C_6$-Alkylthio-$C_2$ $C_4$-alkyl, Cycloalkyl mit 3 bis 6 Ringatomen, das durch 1 bis 2 Sauerstoff- und/oder Schwefelatome unterbrochen sein kann oder gegebenenfalls durch Fluor, Chlor, Brom, Methyl, Ethyl, Propyl, iso-Propyl, Methoxy, Ethoxy, Trifluormethyl oder Nitro substituiertes Aryl steht,

M für Wasserstoff, oder für einen geradkettigen oder verzweigten Rest der Reihe $C_1$-$C_8$-Alkyl, $C_1$-$C_4$-Alkoxyalkyl steht,

oder worin

A und L oder L und M gemeinsam mit den Atomen, an die sie gebunden sind, einen 3 bis 6-gliedrigen Ring bilden, der gesättigt oder einfach ungesättigt sein kann, der durch $C_1$-$C_4$-Alkyl, $C_1$-$C_2$-Alkoxy, durch Trifluormethyl oder Phenyl substituiert sein kann und/oder durch Sauerstoff/Schwefel unterbrochen sein kann,

R für einen gegebenenfalls durch Halogen substituierten geradkettigen oder verzweigten Rest der Reihe $C_1$-$C_8$-Alkyl, $C_3$-$C_4$-Alkenyl, $C_3$-$C_4$-Alkinyl, $C_1$-$C_6$-Alkoxy-$C_2$-$C_4$-alkyl, $C_1$-$C_4$-Polyalkoxy-$C_2$-$C_4$-alkyl, $C_1$-$C_6$-Alkylthio-$C_2$-$C_4$-alkyl, Cycloalkyl mit 3 bis 6 Ringatomen, das durch 1 bis 2 Sauerstoff- und/oder Schwefelatome unterbrochen sein kann, oder für gegebenenfalls durch Fluor, Chlor, Brom, Methyl, Ethyl, Propyl, iso-Propyl, Methoxy, Ethoxy, Trifluormethyl oder Nitro substituiertes Aryl steht,

sowie die enantiomeren Verbindungen der Formel (I) ausgenommen die Verbindungen: 4-Amino-1-cyclopropyl-3-(3-trifluormethylphenyl)-3-pyrrolin-2-on und 4-Amino-1-isopropyl-3-(3-trifluormethylphenyl)-3-pyrrolin-2-on.

Verwendet man gemäß allgemeinem Herstellungsverfahren (aα) 3-(3-Trifluormethylphenyl)-1-isopropyl-5-methylpyrrolidin-2,4-dion und Ammoniumacetat als Ausgangsstoffe, so kann der Verlauf des erfindungsgemäßen Verfahrens durch folgendes Reaktionsschema wiedergegeben werden:

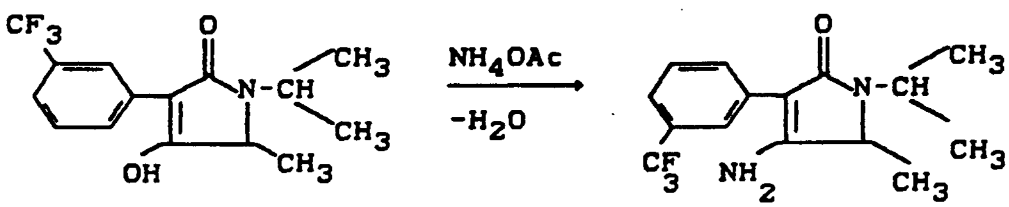


Verwendet man gemäß dem allgemeinen Herstellungsverfahren (aβ) N-(3-Trifluormethylphenylacetyl)-N-methylamino-2-ethyl-essigsäurenitril, so kann der Verlauf des erfindungsgemäßen Verfahrens durch folgendes Reaktionsschema wiedergegeben werden:

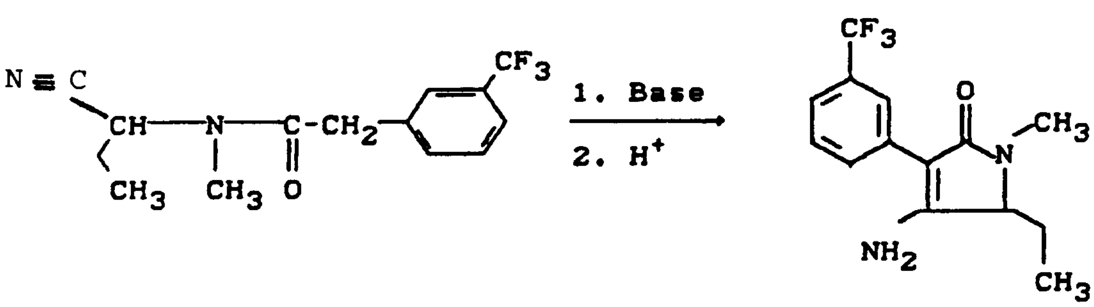

Verwendet man gemäß allgemeinem Herstellungsverfahren (bα) 3-(3,4-Dichlorphenyl)-1-(4-chlorphenyl)-4-amino-3-pyrrolin-2-on und Acetylchlorid als Ausgangsstoffe, so kann der Verlauf des erfindungsgemäßen Verfahrens durch folgendes Reaktionsschema wiedergegeben werden:

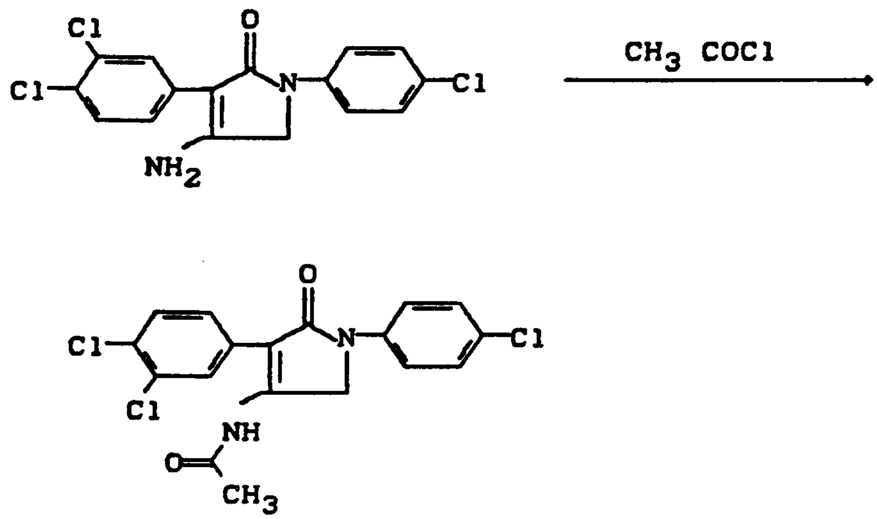


Verwendet man gemäß allgemeinem Herstellungsverfahren (bβ) 3-(3-Methoxyphenyl)-1-phenyl-5-methyl-4-amino-3-pyrrolin-2-on und 2-Ethyl-buttersäure-anhydrid als Ausgangsstoffe, so kann der Verlauf des erfindungsgemäßen Verfahrens durch folgendes Reaktionsschema wiedergegeben werden:

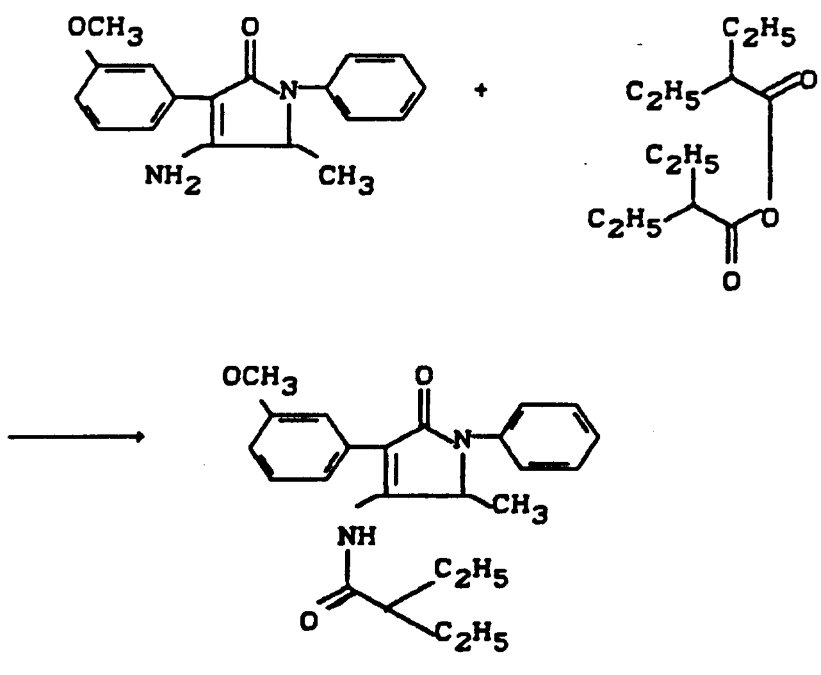

Verwendet man gemäß allgemeinem Herstellungsverfahren (c) 3-(3-Trifluormethylphenyl)-1-tert.-butyl-5-methyl-4-amino-3-pyrrolin-2-on und Chlorameisensäuremethylester als Ausgangsstoffe, so kann der Verlauf des erfindungsgemäßen Verfahrens durch folgendes Reaktionsschema wiedergegeben werden:

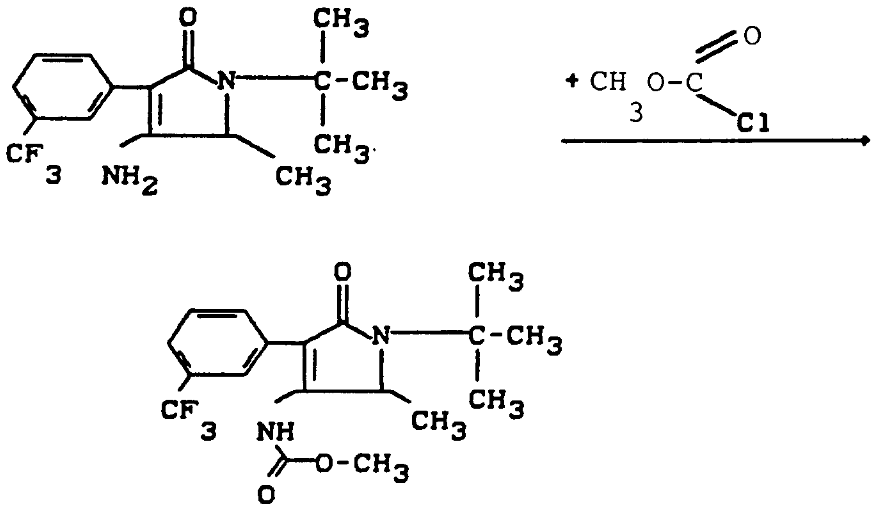

Die zur Durchführung des erfindungsgemäßen Verfahrens (a$\alpha$) als Ausgangsstoffe benötigten Verbindungen der Formel (II)

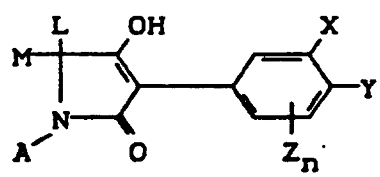

(II)

in welcher
A, L, M, X, Y, Z und n die oben angegebenen Bedeutungen haben,
erhält man, wenn man N-Acylaminosäureester der Formel (VII)

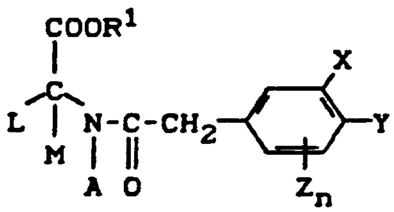

(VII)

in welcher
A, L, M, X, Y, Z und n die oben angegebenen Bedeutungen haben, und
$R^1$ für Alkyl, insbesondere Methyl oder Ethyl steht,
in Gegenwart eines Verdünnungsmittels und in Gegenwart einer Base intramolekular kondensiert.

Die Verbindungen der Formel (VII) sind teilweise bekannt oder lassen sich nach bekannten Methoden in einfacher Weise herstellen.

So erhält man beispielsweise N-Acylaminosäureester der Formel (VII), wenn man Acylaminosäureester der Formel (VIII)

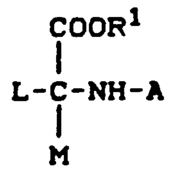

(VIII)

in welcher

$R^1$ für Wasserstoff oder Alkyl, insbesondere Methyl oder Ethyl, steht und

A, L und M die oben angegebenen Bedeutungen haben,

mit Phenylessigsäurehalogeniden der Formel (IX)

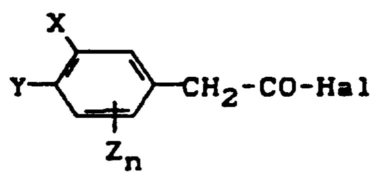

(IX)

in welcher

Hal für Halogen, insbesondere Fluor, Chlor oder Brom, steht und

X, Y, Z und n die oben angegebenen Bedeutungen haben,

in üblicher Weise acyliert (vgl. Chem. Rev. 52 (1953) 237-416 und Organikum, 9. Auflage, 446 (1970), VEB Deutscher Verlag der Wissenschaften),

oder wenn man N-Acylaminosäuren der Formel (II), in welcher $R^1$ für Wasserstoff steht, in üblicher Weise verestert (vgl. Chem. Ind. (London) 1568 (1968)).

Verbindungen der Formel (VII), in denen $R^1$ für Wasserstoff steht, lassen sich beispielsweise auch aus Phenylessigsäurehalogeniden der Formel (IX) und Aminosäuren herstellen.

Verbindungen der Formel (VIII) sind nach literaturbekannten Verfahren aus $\alpha$-Halogencarbonsäuren bzw. -estern und Aminen bzw. Anilinen erhältlich (Advanced Organic Chemistry, J. March, S. 377, Mc Graw-Hill Inc. (1977)).

Die Phenylessigsäurehalogenide der Formel (IX) sind bekannte Verbindungen der organischen Chemie.

Verbindungen der Formel (II) sind teilweise bekannte Verbindungen aus EP-A-0 415 185. Nicht bekannte Verbindungen sind ebenfalls Teil des Anspruchs (siehe Tabelle 1).

Tabelle 1

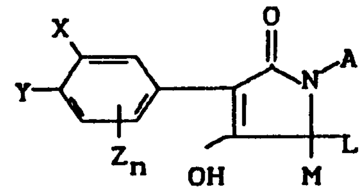

(II)

| Bsp.Nr. | X | Y | $Z_n$ | M | L | A |
|---|---|---|---|---|---|---|
| 1 | $CF_3$ | H | - | H | H | 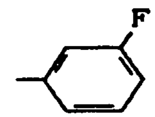 |
| 2 | $CF_3$ | H | - | $CH_3$ | H | $-CH_2-CH_2OCH_3$ |
| 3 | $CF_3$ | H | - | $CH_3$ | H | $i-C_3H_7$ |
| 4 | $CF_3$ | H | - | $CH_3$ | H | $CH_3$ |
| 5 | H | H | - | $CH_3$ | H | $i-C_3H_7$ |
| 6 | $CF_3$ | H | - | $CH_3$ | H | 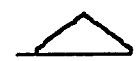 |
| 7 | $CF_3$ | H | - | $CH_3$ | H | $C_2H_5$ |
| 8 | $CF_3$ | H | - | $CH_3$ | H | 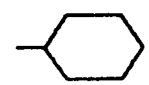 |
| 9 | $CF_3$ | H | - | $CH_3$ | H | 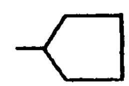 |

**Tabelle 1** (Fortsetzung)

| Bsp.Nr. | X | Y | $Z_n$ | M | L | A |
|---|---|---|---|---|---|---|
| 10 | $CF_3$ | H | - | $CH_3$ | H | 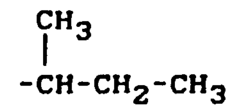 |
| 11 | $CF_3$ | H | - | $CH_3$ | H | 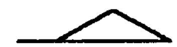 |
| 12 | $CF_3$ | H | - | H | $-CH_2-CH_2-CH_2-CH_2-$ | |
| 13 | H | Cl | - | H | $-CH_2-S-CH_2-CH_2-$ | |
| 14 | H | Cl | - | H | $-CH_2-S-CH_2-$ | |
| 15 | H | Cl | - | H | $-CH_2-CH_2-S-CH_2-$ | |
| 16 | Cl | Cl | - | H | $CH_3$ | $CH_3$ |
| 17 | Cl | Cl | - | H | $-CH_2-S-CH_2-CH_2-$ | |
| 18 | Cl | Cl | - | H | $CH_3$ | $i-C_3H_7$ |
| 19 | Cl | Cl | - | H | $CH_3$ | 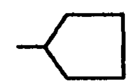 |
| 20 | Cl | Cl | - | H | $C_2H_5$ | $CH_3$ |

**Tabelle 1** (Fortsetzung)

| Bsp.Nr. | X | Y | $Z_n$ | L | M | A |
|---|---|---|---|---|---|---|
| 21 | Cl | Cl | - | H | $CH_3$ | 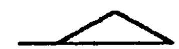 |
| 22 | Cl | Cl | - | H | $CH_3$ | $C_2H_5$ |
| 23 | Cl | Cl | - | H | $CH_3$ | $i-C_4H_9$ |
| 24 | Br | H | - | H | $CH_3$ | $CH_3$ |
| 25 | $CF_3$ | H | - | H | $CH_3$ | $C_6H_5$ |
| 26 | $CF_3$ | H | - | H | $CH_3$ | 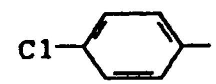 |
| 27 | $CF_3$ | H | - | H | $CH_3$ | 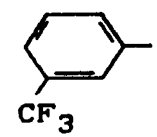 |
| 28 | $CF_3$ | H | - | H | $CH_3$ | 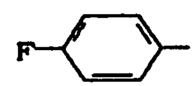 |
| 29 | $CF_3$ | H | - | H | $CF_3$ | 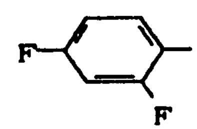 |

**Tabelle 1** (Fortsetzung)

| Bsp.Nr. | X | Y | $Z_n$ | M | L | A |
|---|---|---|---|---|---|---|
| 30 | H | 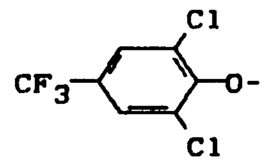 | - | H | $CH_3$ | $C_6H_5$- |
| 31 | 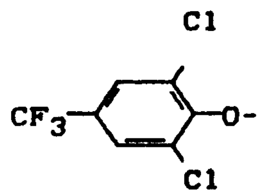 | H | - | H | $CH_3$ | $C_6H_5$- |
| 32 | H | H | - | H | $CH_3$ | 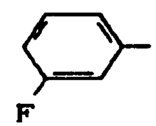 |
| 33 | $CF_3$ | Cl | - | H | $-(CH_2)_4-$ | |
| 34 | $CF_3$ | H | - | H | $CH_3$ | $H_3CO-CH_2-CHCH_3$- |
| 35 | $CF_3$ | H | - | H | 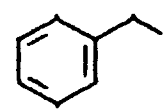 | $CH_3$ |
| 36 | $CF_3$ | H | - | H | 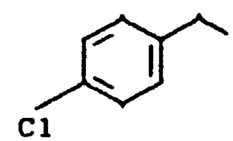 | $CH_3$ |

<u>Tabelle 1</u> (Fortsetzung)

| Bsp.Nr. | X | Y | $Z_n$ | M | L | A |
|---|---|---|---|---|---|---|
| 37 | $CF_3$ | H | - | H | 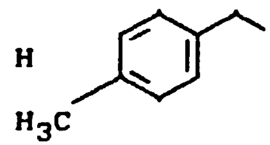 | $CH_3$ |
| 38 | $CF_3$ | H | - | H | 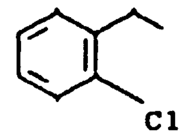 | $CH_3$ |
| 39 | $CF_3$ | H | - | H | 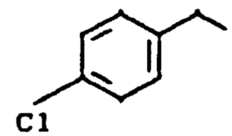 | $CH_3$ |
| 40 | $CF_3$ | H | - | H | 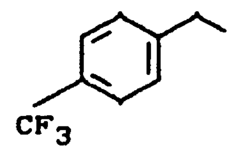 | $CH_3$ |
| 41 | $CF_3$ | H | - | H | $C_6H_5$ | $CH_3$ |
| 42 | $CF_3$ | H | - | H | 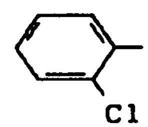 | $CH_3$ |

**Tabelle 1** (Fortsetzung)

| Bsp.Nr. | X | Y | $Z_n$ | M | L | A |
|---|---|---|---|---|---|---|
| 43 | $CF_3$ | H | - | H | 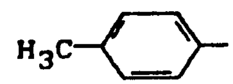 | $CH_3$ |
| 44 | $CF_3$ | H | - | H | 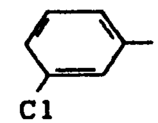 | $CH_3$ |
| 45 | $CF_3$ | H | - | H | 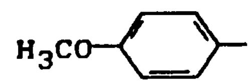 | $CH_3$ |
| 46 | $CF_3$ | H | - | H | 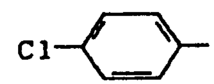 | $CH_3$ |
| 47 | $CF_3$ | H | - | $CH_3$ | H | $CH_2$-iPr |
| 48 | $CF_3$ | H | - | H | $C_2H_5$ | $C_2H_5$ |
| 49 | $CF_3$ | H | - | $CH_3$ | H | $-CH(CH_3)_2$ |

**Tabelle 1** (Fortsetzung)

| Bsp.Nr. | X | Y | $Z_n$ | M | L | A |
|---|---|---|---|---|---|---|
| 50 | $CF_3$ | H | - | $CH_3$ | H | $-C(CH_3)_2-CH_2-C(CH_3)_3$ |
| 51 | $CF_3$ | H | - | $CH_3$ | H | 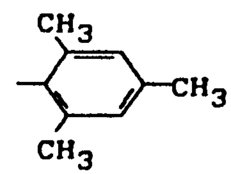 |
| 52 | $CF_3$ | H | - | $CH_3$ | H | 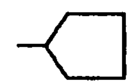 |
| 53 | $CF_3$ | H | - | $CH_3$ | H | $-CH_2CH_2-O-CH_3$ |
| 54 | $CF_3$ | H | - | $CH_3$ | H | 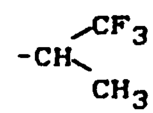 |
| 55 | $CF_3$ | H | - | $CH_3$ | H | 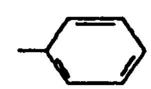 |

**Tabelle 1** (Fortsetzung)

| Bsp.Nr. | X | Y | $Z_n$ | M | L | A |
|---|---|---|---|---|---|---|
| 56 | $CF_3$ | H | - | $CH_3$ | H | 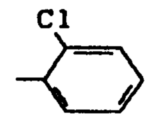 |
| 57 | $CF_3$ | H | - | $CH_3$ | H | 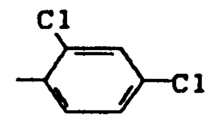 |
| 58 | $CF_3$ | H | - | $CH_3$ | H | 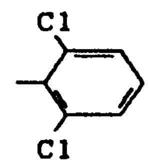 |
| 59 | $CF_3$ | H | - | $CH_3$ | H | 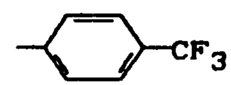 |
| 60 | $CF_3$ | H | - | $CH_3$ | H | 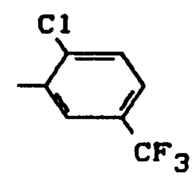 |

**Tabelle 1** (Fortsetzung)

| Bsp.Nr. | X | Y | $Z_n$ | M | L | A |
|---|---|---|---|---|---|---|
| 61 | $CF_3$ | H | - | $CH_3$ | H | 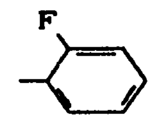 |
| 62 | $CF_3$ | H | - | $CH_3$ | H | 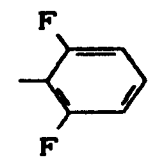 |
| 63 | $CF_3$ | H | - | $CH_3$ | H | $-CH_2-C(CH_3)_3$ |
| 64 | $CF_3$ | H | - | $CH_3$ | H | $-CH(CH_3)-C(CH_3)_3$ |
| 65 | $CF_3$ | H | - | $CH_3$ | H | $-CH_2$ 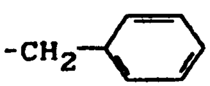 |
| 66 | H | H | - | $CH_3$ | H | 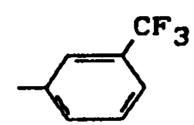 |

**Tabelle 1** (Fortsetzung)

| Bsp.Nr. | X | Y | $Z_n$ | M | L | A |
|---|---|---|---|---|---|---|
| 67 | H | H | - | $CH_3$ | H | 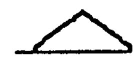 |
| 68 | $CF_3$ | H | - | $CH_3$ | H | $-CH(CH_3)-CH(CH_3)_2$ |
| 69 | $CF_3$ | H | - | $CH_3$ | H | 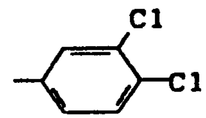 |
| 70 | 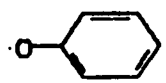 | H | - | $CH_3$ | H | 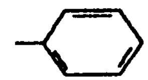 |
| 71 | H | 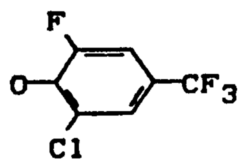 | - | $CH_3$ | H | 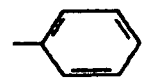 |
| 72 | $CF_3$ | H | - | $CH_3$ | H | 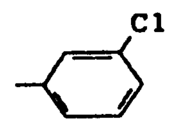 |

**Tabelle 1** (Fortsetzung)

| Bsp.Nr. | X | Y | $Z_n$ | M | L | A |
|---|---|---|---|---|---|---|
| 73 | H | H | - | $CH_3$ | H | 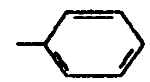 |
| 74 | H | H | - | $CH_3$ | H | 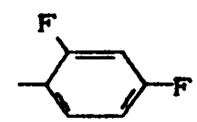 |
| 75 | $CF_3$ | H | - | $CH_3$ | H | 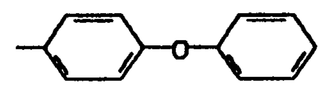 |
| 76 | $CF_3$ | H | - | $CH_3$ | H | 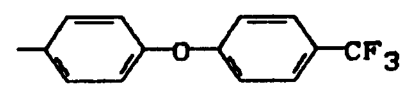 |
| 77 | H | H | - | $CH_3$ | H | 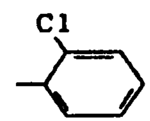 |

<u>Tabelle 1</u> (Fortsetzung)

| Bsp.Nr. | X | Y | $Z_n$ | M | L | A |
|---|---|---|---|---|---|---|
| 78 | F | F | - | $CH_3$ | H | 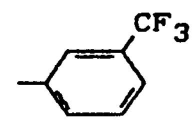 |
| 79 | $CF_3$ | H | - | $CH_3$ | H | 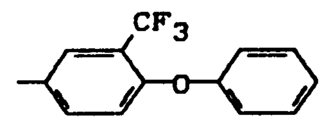 |
| 80 | Cl | Cl | - | $CH_3$ | H | $-CH_2-CH(CH_3)_2$ |
| 81 | Cl | Cl | - | $CH_3$ | H | $-CH-C_2H_5$<br>\|<br>$CH_3$ |
| 82 | Cl | Cl | - | $CH_3$ | H | 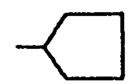 |
| 83 | Cl | Cl | - | $CH_3$ | H | 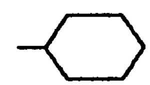 |

**Tabelle 1** (Fortsetzung)

| Bsp.Nr. | X | Y | $Z_n$ | M | L | A |
|---|---|---|---|---|---|---|
| 84 | Cl | Cl | - | $CH_3$ | H | $-CH_2CH_2-O-CH_3$ |
| 85 | Cl | Cl | - | $CH_3$ | H | $-CH(CH_3)_2$ |
| 86 | Cl | Cl | - | $CH_3$ | H | $-CH_3$ |
| 87 | Br | H | - | $CH_3$ | H | $-CH_2-CH(CH_3)_2$ |
| 88 | Br | H | - | $CH_3$ | H | $-CH(CH_3)-C_2H_5$ |
| 89 | Br | H | - | $CH_3$ | H | 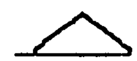 |
| 90 | Br | H | - | $CH_3$ | H | 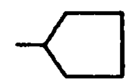 |
| 91 | Br | H | - | $CH_3$ | H | 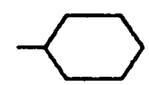 |
| 92 | Br | H | - | $CH_3$ | H | $-CH_2CH_2-O-CH_3$ |

**Tabelle 1** (Fortsetzung)

| Bsp.Nr. | X | Y | $Z_n$ | M | L | A |
|---|---|---|---|---|---|---|
| 93 | Br | H | - | $CH_3$ | H | $-CH(CH_3)_2$ |
| 94 | Br | H | - | $CH_3$ | H | $-C_2H_5$ |
| 95 | Br | H | - | $CH_3$ | H | $-CH_3$ |

Das Verfahren zur Herstellung von Verbindungen der Formel (II) ist dadurch gekennzeichnet, daß man Verbindungen der Formel (VII) in Gegenwart von Basen einer intramolekularen Kondensation unterwirft.

Als Verdünnungsmittel können bei diesem erfindungsgemäßen Verfahren alle üblichen inerten organischen Solventien eingesetzt werden. Vorzugsweise verwendbar sind Kohlenwasserstoffe, wie Toluol und Xylol, ferner Ether, wie Dibutylether, Tetrahydrofuran, Dioxan, Glylkoldimethylether und Diglykoldimethylether, außerdem polare Lösungsmittel, wie Dimethylsulfoxid, Sulfolan, Dimethylformamid, N-Methyl-pyrrolidon, sowie Alkohole wie Ethanol, Methanol, Isobutanol, sek.-Butanol, tert.-Butanol.

Als Deprotonierungsmittel können bei der Durchführung dieses erfindungsgemäßen Verfahrens alle üblichen Protonenakzeptoren eingesetzt werden. Vorzugsweise verwendbar sind Alkalimetall- und Erdalkalimetall-oxide, -hydroxide und -carbonate, wie Natriumhydroxid, Kaliumhydroxid, Magnesiumoxid, Calciumoxid, Natriumcarbonat, Kaliumcarbonat und Calciumcarbonat, die auch in Gegenwart von Phasentransferkatalysatoren wie z.B. Triethylbenzylammoniumchlorid, Tetrabutylammoniumbromid, Adogen 464 oder TDA 1 eingesetzt werden können. Ferner sind Alkalimetall- und Erdalkalimetallamide und -hydride, wie

Adogen 464 = Methyltrialkyl($C_8$-$C_{10}$)ammoniumchlorid
TDA 1 = Tris(methoxyethoxylethyl)amin

Natriumamid, Natriumhydrid und Calciumhydrid, und außerdem auch Alkalimetall-alkoholate, wie Natrium-methylat und Kalium-tert.-butylat einsetzbar.

Die Reaktionstemperaturen können bei der Durchführung dieses erfindungsgemäßen Verfahrens innerhalb eines größeren Bereiches variiert werden. Im allgemeinen arbeitet man bei Temperaturen zwischen 0 °C und 250 °C, vorzugsweise zwischen 50 °C und 150 °C.

Das erfindungsgemäße Verfahren wird im allgemeinen unter Normaldruck durchgeführt.

Bei der Durchführung dieses erfindungsgemäßen Verfahrens setzt man die Reaktionskomponenten der Formel (VII) und die deprotonierenden Basen im allgemeinen in etwa äquimolaren Mengen ein. Es ist jedoch auch möglich, die eine oder andere Komponente in einem größeren Überschuß (bis zu 3 Mol) zu verwenden.

Als Verdünnungsmittel zur Durchführung des erfindungsgemäßen Verfahrens (aα) kommen vorzugsweise inerte organische Lösungsmittel in Frage. Hierzu gehören insbesondere aliphatische, alicyclische oder aromatische, gegebenenfalls halogenierte Kohlenwasserstoffe, wie beispielsweise Benzin, Ligroin, Benzol, Toluol, Xylol, Chlorbenzol, Petrolether, Pentan, Hexan, Heptan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether, Ketone wie Aceton oder Butanon, Nitrile wie Acetonitril oder Propionitril, Amide, wie Dimethylformamid, Dimethylacetamid, N-Methylformanilid, N-Methylpyrrolidon oder Hexamethylphosphorsäuretriamid oder Ester, wie Essigsäureethylester. Die Amine der Formel (IV) in flüssiger Form können in entsprechendem Überschuß ebenfalls als Lösungsmittel eingesetzt werden.

Das erfindungsgemäße Verfahren (aα) wird gegebenenfalls in Gegenwart eines wasserentziehenden Mittels durchgeführt. Vorzugsweise verwendet man als wasserentziehendes Mittel
Molekularsiebe oder - in katalytischen Mengen - z.B. p-Toluolsulfonsäure. Als günstige Verfahrensweise stellt sich das azeotrope Abdestillieren des Wassers während der Reaktion dar.

Die Reaktionstemperaturen können bei der Durchführung des erfindungsgemäßen Verfahrens (aα) in einem größeren Bereich variiert werden. Im allgemeinen arbeitet man bei Temperaturen zwischen 0 °C und 300 °C, vorzugsweise bei Temperaturen zwischen 20 °C und 250 °C.

Das erfindungsgemäße Verfahren (aα) wird üblicherweise unter Normaldruck durchgeführt. Es ist jedoch auch möglich unter erhöhtem Druck zu arbeiten.

Zur Durchführung des erfindungsgemäßen Verfahrens (aα) setzt man pro Mol in Verbindung der Formel (II) in der B für Hydroxy steht, im allgemeinen 1 bis 30 Mol, vorzugsweise 1 bis 10 Mol Ammoniak oder eines Ammoniumsalzes und gegebenenfalls 1 bis 5 Mol wasserentziehendes Mittel ein. Die Reaktionsdurchführung, Aufarbeitung und Isolierung der Reaktionsprodukte erfolgt in Analogie zu allgemein bekannten Verfahren.

Das Verfahren (aβ) ist dadurch gekennzeichnet, daß man Verbindungen der Formel (III) in Gegenwart von Basen einer intramolekularen Kondensation unterwirft.

Als Verdünnungsmittel können bei dem erfindungsgemäßen Verfahren (aβ) alle üblichen inerten organischen Solventien eingesetzt werden. Vorzugsweise verwendbar sind Kohlenwasserstoffe, wie Toluol und Xylol, ferner Ether, wie Dibutylether, Tetrahydrofuran, Dioxan, Glykoldimethylether und Diglykoldimethylether, außerdem polare Lösungsmittel, wie Dimethylsulfoxid, Sulfolan, Dimethylformamid, N-Methyl-pyrrolidon, sowie Alkohole wie Ethanol, Methanol, Isobutanol, sek.-Butanol, tert.-Butanol.

Als Deprotonierungsmittel können bei der Durchführung des erfindungsgemäßen Verfahrens (aβ) alle üblichen Protonenakzeptoren eingesetzt werden. Vorzugsweise verwendbar sind Alkalimetall- und Erdalkalimetall-oxide, -hydroxide und -carbonate, wie Natriumhydroxid, Kaliumhydroxid, Magnesiumoxid, Calciumoxid, Natriumcarbonat, Kaliumcarbonat und Calciumcarbonat, die auch in Gegenwart von Phasentransferkatalysatoren wie z.B. Triethylbenzylammoniumchlorid, Tetrabutylammoniumbromid, Adogen 464 oder TDA 1 eingesetzt werden können. Ferner sind Alkalimetall- und Erdalkalimetallamide und -hydride, wie Natriumamid, Natriumhydrid und Calciumhydrid, und außerdem auch Alkalimetall-alkoholate, wie Natriummethylat und Kalium-tert.-butylat einsetzbar.

Die Reaktionstemperaturen können bei der Durchführung des erfindungsgemäßen Verfahrens (aβ) innerhalb eines größeren Bereiches variiert werden. Im allgemeinen arbeitet man bei Temperaturen zwischen 0 °C und 250 °C, vorzugsweise zwischen 50 °C und 150 °C.

Das erfindungsgemäße Verfahren (aβ) wird im allgemeinen unter Normaldruck durchgeführt.

Bei der Durchführung des erfindungsgemäßen Verfahrens (aβ) setzt man die Reaktionskomponenten der Formel (III) und die deprotonierenden Basen im allgemeinen in etwa äquimolaren Mengen ein. Es ist jedoch auch möglich, die eine oder andere Komponente in einem größeren Überschuß (bis zu 3 Mol) zu verwenden.

| | |
|---|---|
| Adogen 464 | = Methyltrialkyl)$C_8$-$C_{10}$)ammoniumchlorid |
| TDA 1 | = Tris(methoxyethoxylethyl)amin |

Das Verfahren (bα) ist dadurch gekennzeichnet, daß man Verbindungen der Formel (Ia) mit Carbonsäurehalogeniden der Formel (IV) umsetzt.

Als Verdünnungsmittel können bei dem erfindungsgemäßen Verfahren (bα) bei Verwendung der Säurehalogenide alle gegenüber diesen Verbindungen inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenstoff, Chlorbenzol und o-Dichlorbenzol, außerdem Ketone, wie Aceton und Methylisopropylketon, weiterhin Ether, wie Diethylether, Tetrahydrofuran und Dioxan, darüber hinaus Sulfoxide, wie Dimethylsulfoxid und Sulfolan. Wenn die Hydrolysestabilität des Säurehalogenids es zuläßt, kann die Umsetzung auch in Gegenwart von Wasser durchgeführt werden. Es ist jedoch auch möglich, ohne Solvens zu arbeiten.

Verwendet man die entsprechenden Carbonsäurehalogenide, so kommen als Säurebindemittel bei der Umsetzung nach dem erfindungsgemäßen Verfahren (bα) alle üblichen Säureakzeptoren in Betracht. Vorzugsweise verwendbar sind tertiäre Amine, wie Triethylamin, Pyridin, Diazabicyclooctan (DABCO), Diazabicycloundecen (DBU), Diazabicyclononen (DBN), Hünig-Base und N,N-Dimethylanilin, ferner Erdalkalimetalloxide, wie Magnesium- und Calciumoxid, außerdem Alkali- und Erdalkalimetallcarbonate, wie Natriumcarbonat, Kaliumcarbonat und Calciumcarbonat. Es kann jedoch auch auf Säurebindemittel verzichtet werden.

Die Reaktionstemperaturen können bei dem erfindungsgemäßen Verfahren (aα) auch bei der Verwendung von Carbonsäurehalogeniden innerhalb eines größeren Bereiches variiert werden. Im allgemeinen arbeitet man bei Temperaturen zwischen -20 °C und +200 °C, vorzugsweise zwischen 0 °C und 150 °C.

Bei der Durchführung des erfindungsgemäßen Verfahrens (bα) werden die Ausgangsstoffe der Formel (Ia) und das Carbonsäurehalogenid der Formel (IV) im allgemeinen in angenähert äquivalenten Mengen verwendet. Es ist jedoch auch möglich, das Carbonsäurechlorid in einem größeren Überschuß (bis zu 20 Mol) einzusetzen. Die Aufarbeitung erfolgt nach üblichen Methoden.

Das Verfahren (bβ) ist dadurch gekennzeichnet, daß man Verbindungen der Formel (Ia) mit Carbonsäurehydriden der Formel (V) umsetzt.

Verwendet man bei dem erfindungsgemäßen Verfahren (bβ) als Reaktionskomponente der Formel (V) Carbonsäureanhydride, so können als Verdünnungsmittel vorzugsweise diejenigen Verdünnungsmittel verwendet werden, die auch bei der Verwendung von Säurehalogeniden vorzugsweise in Betracht kommen. Im übrigen kann auch ein im Überschuß eingesetztes Carbonsäurehydrid gleichzeitig als Verdünnungsmittel fungieren.

Die Reaktionstemperaturen können bei dem erfindungsgemäßen Verfahren (bβ) auch bei der Verwendung von Carbonsäureanhydriden innerhalb eines größeren Bereiches variiert werden. Im allgemeinen arbeitet man bei Temperaturen zwischen -20 °C und +200 °C, vorzugsweise zwischen 0 °C und 150 °C.

Bei der Durchführung des erfindungsgemäßen Verfahrens (bβ) werden die Ausgangsstoffe der Formel (Ia) und das Carbonsäureanhydrid der Formel (V) im allgemeinen in angenähert äquivalenten Mengen verwendet. Es ist jedoch auch möglich, das Carbonsäureanhydrid in einem größeren Überschuß (bis zu 20 Mol) einzusetzen. Die Aufarbeitung erfolgt nach üblichen Methoden.

Im allgemeinen geht man so vor, daß man Verdünnungsmittel und im Überschuß vorhandenes Carbonsäureanhydrid sowie die entstehende Carbonsäure durch Destillation oder durch Waschen mit einem organischen Lösungsmittel oder mit Wasser entfernt.

Das Verfahren (c) ist dadurch gekennzeichnet, daß man Verbindungen der Formel (Ia) mit Chlorameisensäureestern der Formel (VI) umsetzt.

Verwendet man die entsprechenden Chlorameisensäureester, so kommen als Säurebindemittel bei der Umsetzung nach dem erfindungsgemäßen Verfahren (c) alle üblichen Säureakzeptoren in Betracht. Vorzugsweise verwendbar sind tertiäre Amine, wie Triethylamin, Pyridin, DABCO, DBU, DBN, Hünig-Base und N,N-Dimethyl-anilin, ferner Erdalkalimetalloxide, wie Magnesium- und Calcium-oxid, außerdem Alkali- und Erdalkali-metall-carbonate, wie Natriumcarbonat, Kaliumcarbonat und Calciumcarbonat.

Als Verdünnungsmittel können bei dem erfindungsgemäßen Verfahren (c) bei Verwendung der Chlorameisensäureester alle gegenüber diesen Verbindungen inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenwasserstoff, Chlorbenzol und o-Dichlorbenzol, außerdem Ketone, wie Aceton und Methylisopropylketon, weiterhin Ether, wie Diethylether, Tetrahydrofuran und Dioxan, darüberhinaus Carbonsäureester, wie Ethylacetat, und auch stark polare Solventien, wie Dimethylsulfoxid und Sulfolan. Es ist jedoch auch möglich, ohne Solvens zu arbeiten.

Bei Verwendung der Chlorameisensäureester als Carbonsäure-Derivate der Formel (VI) können die Reaktionstemperaturen bei der Durchführung des erfindungsgemäßen Verfahrens (c) innerhalb eines größeren Bereiches variiert werden. Arbeitet man in Gegenwart eines Verdünnungsmittels und eines Säurebindemittels, so liegen die Reaktionstemperaturen im allgemeinen zwischen -20 °C und +250 °C, vorzugsweise zwischen 0 °C und 150 °C.

Das erfindungsgemäße Verfahren (c) wird im allgemeinen unter Normaldruck durchgeführt.

Bei der Durchführung des erfindungsgemäßen Verfahrens (c) werden die Ausgangsstoffe der Formel (Ia) und der entsprechende Chlorameisensäureester der Formel (VI) im allgemeinen in angenähert äquivalenten Mengen verwendet. Es ist jedoch auch möglich, die eine oder andere Komponente in einem größeren Überschuß (bis zu 10 Mol) einzusetzen. Die Aufarbeitung erfolgt dann nach üblichen Methoden. Im allgemeinen geht man so vor, daß man ausgefallene Salze entfernt und das verbleibende Reaktionsgemisch durch Entfernen des Verdünnungsmittels im Vakuum einengt.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewandten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Die erfindungsgemäßen Wirkstoffe eignen sich sehr gut zur selektiven Bekämpfung von mono- und dikotylen Unkräutern in mono- und dikotylen Kulturen im Vor- und Nachauflaufverfahren.

Darüber hinaus zeigen die erfindungsgemäßen Verbindungen auch eine blattinsektizide Wirkung.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl,

Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide infrage, beispielsweise Anilide, wie z.B. Diflufenican und Propanil; Arylcarbonsäuren, wie z.B. Dichlorpicolinsäure, Dicamba und Picloram; Aryloxyalkansäuren, wie z.B. 2,4 D, 2,4 DB, 2,4 DP, Fluoroxypyr, MCPA, MCPP und Triclopyr; Aryloxy-phenoxy-alkansäureester, wie z.B. Diclofop-methyl, Fenoxaprop-ethyl, Fluazifop-butyl, Haloxyfop-methyl und Quizalofop-ethyl; Azinone, wie z.B. Chloridazon und Norflurazon; Carbamate, wie z.B. Chlorpropham, Desmedipham, Phenmedipham und Propham; Chloracetanilide, wie z.B. Alachlor, Acetochlor, Butachlor, Metazachlor, Metolachlor, Pretilachlor und Propachlor; Dinitroaniline, wie z.B. Oryzalin, Pendimethalin und Trifluralin; Diphenylether, wie z.B. Acifluorfen, Bifenox, Fluoroglycofen, Fomesafen, Halosafen, Lactofen und Oxyfluorfen; Harnstoffe, wie z.B. Chlortoluron, Diuron, Fluometuron, Isoproturon, Linuron und Methabenzthiazuron; Hydroxylamine, wie z.B. Alloxydim, Clethodim, Cycloxydim, Sethoxydim und Tralkoxydim; Imidazolinone, wie z.B. Imazethapyr, Imazamethabenz, Imazapyr und Imazaquin; Nitrile, wie z.B. Bromoxynil, Dichlobenil und Ioxynil; Oxyacetamide, wie z.B. Mefenacet; Sulfonylharnstoffe, wie z.B. Amidosulfuron, Bensulfuron-methyl, Chlorimuron-ethyl, Chlorsulfuron, Cinosulfuron, Metsulfuron-methyl, Nicosulfuron, Primisulfuron, Pyrazosulfuron-ethyl, Thifensulfuron-methyl, Triasulfuron und Tribenuron-methyl; Thiolcarbamate, wie z.B. Buylate, Cycloste, Diallate, EPTC, Esprocarb, Molinate, Prosulfocarb, Thiobencarb und Triallate; Triazine, wie z.B. Atrazin, Cyanazin, Simazin, Simetryne, Terbutryne und Terbutylazin; Triazinone, wie z.B. Hexazinon, Metamitron und Metribuzin; Sonstige, wie z.B. Aminotriazol, Benfuresate, Bentazone, Cinmethylin, Clomazone, Clopyralid, Difenzoquat, Dithiopyr, Ethofumesate, Fluorochloridone, Glufosinate, Glyphosate, Isoxaben, Pyridate, Quinchlorac, Quinmerac, Sulphosate und Tridiphane.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 15 kg Wirkstoff pro Hektar Bodenfläche, vorzugsweise zwischen 0,05 und 10 kg pro ha.

Die Herstellung und die Verwendung der erfindungsgemäßen Wirkstoffe geht aus den nachfolgenden Beispielen hervor.

Herstellungsbeispiele

Beispiel 1

Synthese der Acylaminosäureester (VIII)

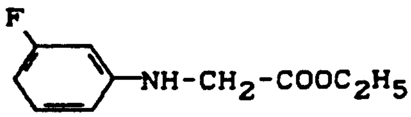

MW 197

3-Fluorphenylacylaminosäure-ethylester

22,2 g (0,2 Mol) 3-Fluoranilin werden in 100 ml Xylol vorgelegt und 20,2 g (0,2 Mol) Triethylamin zugegeben. Sodann kühlt man auf 10 - 20 °C herab und läßt dabei 33,4 g (0,2 Mol) Bromessigsäureethylester zutropfen. Nach Beendigung dieses Vorganges erhitzt man 8 Stunden zum Rückflußsieden, läßt danach abkühlen und entfernt das Xylol im Vakuum. Den Rückstand versetzt man mit 100 ml Wasser, extrahiert zweimal mit je 60 ml Methylenchlorid und trocknet über Magnesiumsulfat. Schließlich filtriert man das Magnesiumsulfat wieder ab, engt die Lösung im Vakuum ein und erhält 37,6 g (95,4 % der Theorie) 3-Fluorphenylacylaminosäureethylester in Form eines gelben Öles, das durch NMR-Spektroskopie identifiziert wird.

Beispiel 2

Synthese der N-Acylaminosäureester (VII)

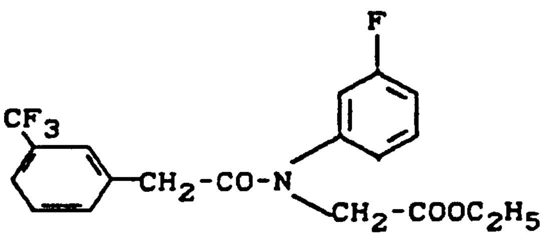

MW 383

N-(3-Fluorphenyl)-N-(3-trifluormethylphenylacetyl)-acylaminosäure-ethylester

19,7 g (0,1 Mol) 3-Fluorphenylacylaminosäure-ethylester werden mit 10,1 g (0,1 Mol) Triethylamin in 150 ml Tetrahydrofuran vorgelegt. Das Gemisch wird auf 2 bis 6 °C herabgekühlt und sodann 22,3 g (0,1 Mol) 3-Trifluormethylphenylessigsäurechlorid in 20 ml Tetrahydrofuran zugetropft.

Nach Beendigung des Vorganges läßt man 30 Minuten bei Raumtemperatur nachrühren, trägt die Reaktionslösung in 700 ml Eiswasser ein, versetzt mit 100 ml 1normaler HCl, extrahiert zweimal mit je 150 ml Methylenchlorid und trocknet die vereinigten organischen Phasen über Magnesiumsulfat.

Nach Abfiltrieren des Magnesiumsulfats entfernt man das Lösungsmittel im Vakuum und erhält 37 g (96,6 % der Theorie) N-(3-Fluorphenyl)-N-(3-trifluormethylphenylacetyl)-acylaminosäure-ethylester, der durch NMR-Spektroskopie identifiziert wird.

Beispiel 3

Synthese der Zwischenprodukte (II)

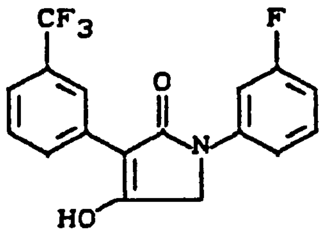

(II-1)

MW 337

3-(3-Trifluormethylphenyl)-1-(3-fluorphenyl)-pyrrolidin-2,4-dion

3,77 g (0,126 Mol) Natriumhydrid werden in 90 ml absolutem Toluol vorgelegt und bei 90 °C bis 100 °C mit 37 g (0,097 Mol) N-(3-Fluorphenyl)-N-(3-trifluormethylphenylacetyl)-acylaminosäure-ethylester, die ebenfalls in 100 ml absolutem Toluol gelöst sind, tropfenweise versetzt. Anschließend läßt man noch eine Stunde unter Rückflußsieden nachreagieren, bevor man auf 3 °C bis 5 °C (Eisbadmischung) herabkühlt und 30 ml Ethanol, 75 ml Wasser und 20 ml konzentrierte Salzsäure zutropft. Den dabei ausfallenden weißen Niederschlag filtriert man schließlich ab, wäscht mit Aceton und trocknet.

Man erhält 27,2 g (83,4 % der Theorie) 3-(3-Trifluormethylphenyl)-1-(3-fluorphenyl)-pyrrolidin-2,4-dion vom Schmelzpunkt 208 °C bis 209 °C, das durch NMR-Spektroskopie identifiziert wird.

In analoger Weise zur oben beschriebenen Synthese der Zwischenprodukte (II) und unter Berücksichtigung der Angaben in der Beschreibung zum erfindungsgemäßen Verfahren (a) erhält man die nachfolgend in Tabelle 2 aufgeführten Produkte der Formel (II)

**Tabelle 2**

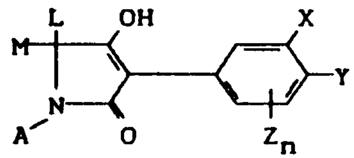

(II)

| Bsp.Nr. | X | Y | Z | n | M | L | A | Schmelzpunkt/° C |
|---|---|---|---|---|---|---|---|---|
| II-2 | $CF_3$ | H | - | - | $CH_3$ | H | $-CH_2-CH_2OCH_3$ | 138 |
| II-3 | $CF_3$ | H | - | - | $CH_3$ | H | $i-C_3H_7$ | 189 |
| II-4 | $CF_3$ | H | - | - | $CH_3$ | H | $CH_3$ | 207 |
| II-5 | H | H | - | - | $CH_3$ | H | $i-C_3H_7$ | 180 |
| II-6 | H | H | - | - | $CH_3$ | H | 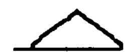 | 207 |
| II-7 | $CF_3$ | H | - | - | $CH_3$ | H | $C_2H_5$ | 205 |
| II-8 | $CF_3$ | H | - | - | $CH_3$ | H | 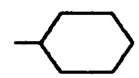 | 218 |
| II-9 | $CF_3$ | H | - | - | $CH_3$ | H | 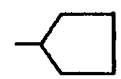 | 191 |

<u>Tabelle 2</u> (Fortsetzung)

| Bsp.Nr. | X | Y | Z | n | M | L | A | Schmelzpunkt/°C |
|---|---|---|---|---|---|---|---|---|
| II-10 | $CF_3$ | H | - | - | $CH_3$ | H | $CH_3$<br>$-CH-CH_2-CH_3$ | 168 |
| II-11 | $CF_3$ | H | - | - | $CH_3$ | H | 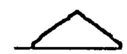 | 218 |
| II-12 | $CF_3$ | H | - | - | H | | $-CH_2-CH_2-CH_2-CH_2-$ | › 220 |
| II-13 | H | Cl | - | - | H | | $-CH_2-S-CH_2-CH_2-$ | 230 |
| II-14 | H | Cl | - | - | H | | $-CH_2-S-CH_2-$ | 183 |
| II-15 | H | Cl | - | - | H | | $-CH_2-CH_2-S-CH_2-$ | 230 |
| II-16 | Cl | Cl | - | - | H | $CH_3$ | $CH_3$ | 233 |
| II-17 | Cl | Cl | - | - | H | | $-CH_2-S-CH_2-CH_2-$ | 230 |
| II-18 | Cl | Cl | - | - | H | $CH_3$ | $i-C_3H_7$ | 202 |
| II-19 | Cl | Cl | - | - | H | $CH_3$ | 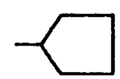 | › 220 |
| II-20 | Cl | Cl | - | - | H | $C_2H_5$ | $CH_3$ | 220 |

**Tabelle 2** (Fortsetzung)

| Bsp.Nr. | X | Y | Z | n | L | M | A | Schmelzpunkt/°C |
|---|---|---|---|---|---|---|---|---|
| II-21 | Cl | Cl | - | - | H | $CH_3$ | 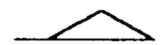 | > 220 |
| II-22 | Cl | Cl | - | - | H | $CH_3$ | $C_2H_5$ | 200 |
| II-23 | Cl | Cl | - | - | H | $CH_3$ | $i\text{-}C_4H_9$ | 196 |
| II-24 | Br | H | - | - | H | $CH_3$ | $CH_3$ | 201 |
| II-25 | $CF_3$ | H | - | - | H | $CH_3$ | $C_6H_5$ | 216 |
| II-26 | $CF_3$ | H | - | - | H | $CH_3$ | 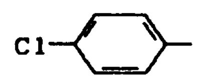 | 112 |
| II-27 | $CF_3$ | H | - | - | H | $CH_3$ | 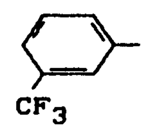 | 201 |
| II-28 | $CF_3$ | H | - | - | H | $CH_3$ | 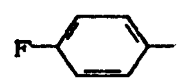 | 195 |

<u>Tabelle 2</u> (Fortsetzung)

| Bsp.Nr. | X | Y | $Z_n$ | L | M | A | Schmelzpunkt/$^0$C |
|---|---|---|---|---|---|---|---|
| II-29 | $CF_3$ | H | - | H | $CH_3$ | 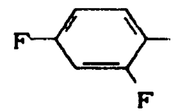 | 216-217 |
| II-30 | H | 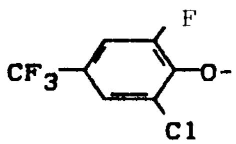 | - | H | $CH_3$ | $C_6H_5$- | 249 |
| II-31 | 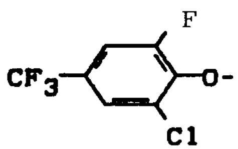 | H | - | H | $CH_3$ | $C_6H_5$ | 77 |
| II-32 | H | H | - | H | $CH_3$ | 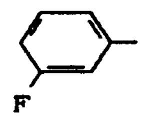 | 239-240 |
| II-33 | $CF_3$ | Cl | - | H | $-(CH_2)_4-$ | | 244 |

<u>Tabelle 2</u> (Fortsetzung)

| Bsp.Nr. | X | Y | $Z_n$ | L | M | A | Schmelzpunkt/°C |
|---|---|---|---|---|---|---|---|
| II-34 | $CF_3$ | H | - | H | $CH_3$ | $H_3CO-CH_2-CHCH_3-$ | 210 |
| II-35 | $CF_3$ | H | - | H | 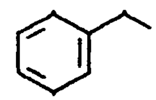 | $CH_3$ | 154-156 |
| II-36 | $CF_3$ | H | - | H | 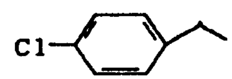 | $CH_3$ | 148-150 |
| II-37 | $CF_3$ | H | - | H | 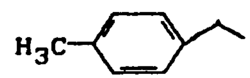 | $CH_3$ | 183-185 |
| II-38 | $CF_3$ | H | - | H | 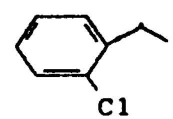 | $CH_3$ | 138-140 |

<u>Tabelle 2</u> (Fortsetzung)

| Bsp.Nr. | X | Y | $Z_n$ | L | M | A | Schmelzpunkt/°C |
|---|---|---|---|---|---|---|---|
| II-39 | $CF_3$ | H | - | H | 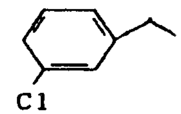 | $CH_3$ | 123-125 |
| II-40 | $CF_3$ | H | - | H | 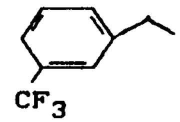 | $CH_3$ | 139-141 |
| II-41 | $CF_3$ | H | - | H | $C_6H_5$ | $CH_3$ | 216-218 |
| II-42 | $CF_3$ | H | - | H | 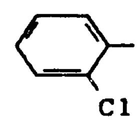 | $CH_3$ | 176-178 |
| II-43 | $CF_3$ | H | - | H | 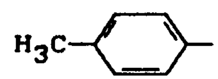 | $CH_3$ | 221-223 |
| II-44 | $CF_3$ | H | - | H | 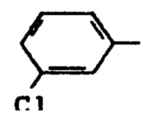 | $CH_3$ | 237-239 |

<u>Tabelle 2</u> (Fortsetzung)

| Bsp.Nr. | X | Y | Z | n | M | L | A | Schmelzpunkt °C |
|---|---|---|---|---|---|---|---|---|
| II-45 | $CF_3$ | H | - | - | H | 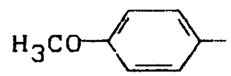 | $CH_3$ | 204-206 |
| II-46 | $CF_3$ | H | - | - | H | 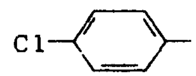 | $CH_3$ | 200-203 |
| II-47 | $CF_3$ | H | - | - | $CH_3$ | H | $CH_2$-iPr | 178 |
| II-48 | $CF_3$ | H | - | - | H | $C_2H_5$ | $C_2H_5$ | 168 |
| II-49 | H | H | - | - | H | H | 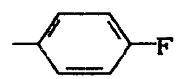 | 250 |
| II-50 | $CF_3$ | H | - | - | H | H | 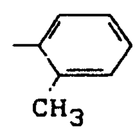 | 223 |

Tabelle 2 (Fortsetzung)

| Bsp.Nr. | X | Y | Z | n | M | L | A | Schmelzpunkt °C |
|---|---|---|---|---|---|---|---|---|
| II-51 | $CF_3$ | H | - | - | H | H | 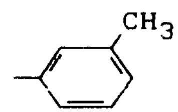 | 248 |
| II-52 | $CF_3$ | H | - | - | H | H | 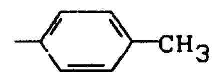 | 250 |
| II-53 | H | $CH_3$ | - | - | H | H | 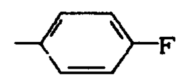 | 250 |
| II-54 | H | F | - | - | H | H | 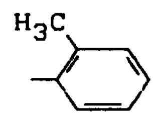 | 250 |
| II-55 | H | F | - | - | H | H | 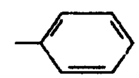 | 250 |

Tabelle 2 (Fortsetzung)

| Bsp.Nr. | X | Y | Z | n | M | L | A | Schmelzpunkt °C |
|---|---|---|---|---|---|---|---|---|
| II-56 | F | H | - | - | H | H | 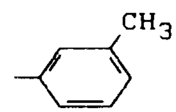 | 248 |
| II-57 | F | H | - | - | H | H | 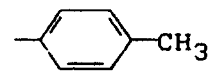 | 248 |
| II-58 | F | H | - | - | H | H | 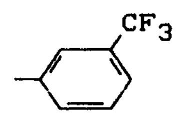 | 247 |
| II-59 | F | H | - | - | H | H | 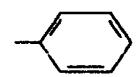 | 235 |
| II-60 | $CF_3$ | H | - | - | H | H | $C_3H_7$-iso | 210 |

<u>Tabelle 2</u> (Fortsetzung)

| Bsp.Nr. | X | Y | Z | n | M | L | A | Schmelzpunkt °C |
|---|---|---|---|---|---|---|---|---|
| II-61 | 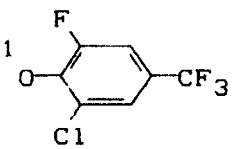 | H | - | - | H | H | 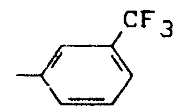 | 205-206 |
| II-62 | 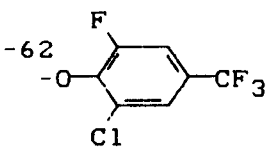 | H | - | - | H | H | 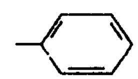 | 226 |
| II-63 | H | H | - | - | H | H | 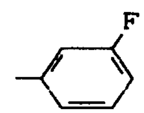 | 230-232 |
| II-64 | $CF_3$ | H | - | - | $CH_3$ | H | 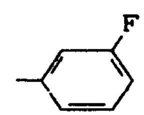 | 208-209 |

Tabelle 2 (Fortsetzung)

| Bsp.Nr. | X | Y | Z | n | M | L | A | Schmelzpunkt °C |
|---|---|---|---|---|---|---|---|---|
| II-65 | Cl | H | - | - | H | H | 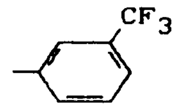 | 235 |
| II-66 | Cl | H | - | - | H | H | 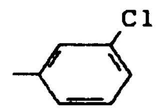 | 243 |
| II-67 | Cl | H | - | - | H | H | 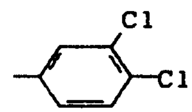 | 250 |
| II-68 | Cl | Cl | - | - | H | H | 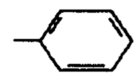 | 265-270 |
| II-69 | $CF_3$ | H | - | - | $CH_3$ | H | $-CH_2CH_2OCH_3$ | 123-124 |
| II-70 | $CF_3$ | H | - | - | $CH_3$ | H | $-CH_2-CH_2-$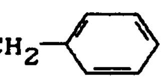 | 189-191 |

<u>Tabelle 2</u> (Fortsetzung)

| Bsp.Nr. | X | Y | Z | n | M | L | A | Schmelzpunkt °C |
|---|---|---|---|---|---|---|---|---|
| II-71 | $CF_3$ | H | - | - | $CH_3$ | H | $-CH_2-$ 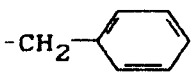 | 123-125 |
| II-72 | $CF_3$ | H | - | - | H | $-CH_2-CH_2-S-CH_2-$ | | 224-226 |
| II-73 | $CF_3$ | H | - | - | H | $-CH_2-CH_2-CH_2-$ | | 234-236 |
| II-74 | $CF_3$ | H | - | - | $CH_3$ | H | $-CH_2-C(CH_3)_3$ | 215-218 |
| II-75 | $CF_3$ | H | - | - | H | 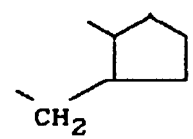 | | 267-270 |
| II-76 | $CF_3$ | H | - | - | - | - | $-CH_2-CH_2-$ 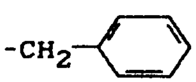 | 215-217 |

<u>Beispiel 4</u>

Synthese der 4-Amino-3-arylpyrrolinon-Derivate der Formel (Ia)

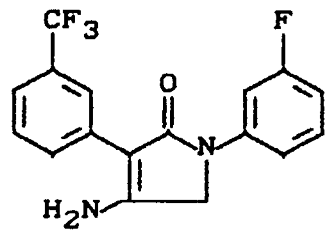

(Ia-1)

MW 336

4-Amino-1-(3-fluorphenyl)-3-(3-trifluormethylphenyl)-pyrrolin-2-on

Verfahren aα

3,77 g (0,01 Mol) 3-(3-Trifluormethylphenyl)-1-(3-fluorphenyl)-pyrrolidin-2,4-dion (II-1) werden in 80 ml Xylol vorgelegt, mit 5 ml Eisessig und 2 ml 25 %iger wäßriger Ammoniaklösung versetzt und anschließend 6 Stunden am Wasserabscheider zum Rückflußsieden erhitzt. Nach jeweils 2 Stunden erfolgt nochmals eine Zugabe von 5 ml Eisessig und 2 ml Ammoniaklösung, so daß schließlich 15 ml Eisessig und 6 ml Ammoniaklösung zugegeben wurden. Nach der letzten Zugabe läßt man noch 2 Stunden am Wasserabscheider unter Rückfluß sieden, bevor man die flüchtigen Bestandteile im Vakuum entfernt. Den Rückstand nimmt man in 50 ml Wasser auf, extrahiert dreimal mit je 80 ml Methylenchlorid, wäscht die vereinigten organischen Phasen einmal mit Wasser, trocknet über Magnesiumsulfat und engt im Vakuum ein. Den dabei anfallenden kristallinen Rückstand verrührt man mit Petrolether/Methylenchlorid (1:1) und filtriert den weißen Niederschlag ab.

Man erhält 3,2 g (73 % der Theorie) 4-Amino-1-(3-fluorphenyl)3-(3-trifluorphenyl)-pyrrolin-2-on mit Schmelzpunkt 201 - 203 °C.

Die Verbindung wird durch NMR und MS-Spektroskopie identifiziert. In Einzelfällen muß das Rohprodukt über Kieselgel mit Toluol/Aceton (1:1) als Laufmittel chromatographiert werden, was die Ausbeuten auf 45 bis 75 % absenken kann.

In analoger Weise zu Beispiel 4 und unter Berücksichtigung der Angaben in der Beschreibung zum erfindungsgemäßen Verfahren (a) erhält man die nachfolgend in Tabelle 3 aufgeführten Endprodukte der Formel (Ia), in denen B für Wasserstoff steht.

Tabelle 3

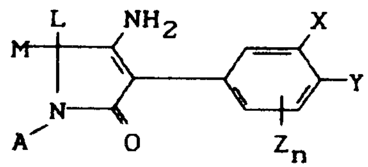

(Ia)

| Bsp.Nr. | X | Y | Z | n | M | L | A | Schmelzpunkt/°C |
|---|---|---|---|---|---|---|---|---|
| Ia-2 | $CF_3$ | H | - | - | $CH_3$ | H | $CH_3$ | 130 |
| Ia-3 | H | H | - | - | $CH_3$ | H | 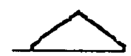 | 190 |
| Ia-4 | H | H | - | - | $CH_3$ | H | i-$C_3H_7$ | 170 |
| Ia-5 | $CF_3$ | H | - | - | H | H | $CH_3$ | 167 |
| Ia-6 | $CF_3$ | H | - | - | $CH_3$ | H | $C_2H_5$ | 180 |
| Ia-7 | $CF_3$ | H | - | - | $C_2H_5$ | H | $C_2H_5$ | 139 |
| Ia-8 | $CF_3$ | H | - | - | $CH_3$ | H | i-$C_3H_7$ | 173 |
| Ia-9 | $CF_3$ | H | - | - | $CH_3$ | H | 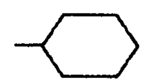 | 160 |
| Ia-10 | $CF_3$ | H | - | - | $CH_3$ | H | i-$C_4H_9$ | 173 |

Tabelle 3 (Fortsetzung)

| Bsp.Nr. | X | Y | Z | n | M | L | A | Schmelzpunkt/°C |
|---|---|---|---|---|---|---|---|---|
| Ia-11 | $CF_3$ | H | - | - | $CH_3$ | H | 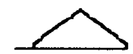 | 145 |
| Ia-12 | $CF_3$ | H | - | - | H | H | 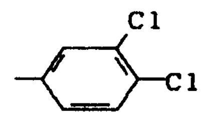 | 236 |
| Ia-13 | $CF_3$ | H | - | - | $CH_3$ | H | 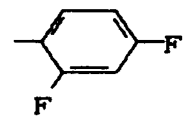 | 238-239 |
| Ia-14 | $CF_3$ | H | - | - | H | H | 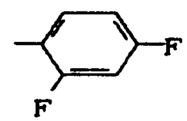 | 185-186 |
| Ia-15 | 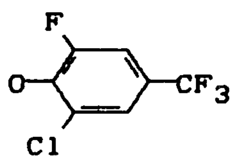 | H | - | - | H | H | 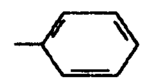 | 110 |
| Ia-16 | $CF_3$ | H | - | - | $CH_3$ | H | 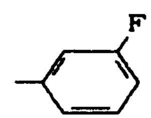 | 143 |

<u>Tabelle 3</u> (Fortsetzung)

| Bsp.Nr. | X | Y | Z | n | M | L | A | Schmelzpunkt/°C |
|---|---|---|---|---|---|---|---|---|
| Ia-17 | Cl | Cl | - | - | $CH_3$ | H | $CH_3$ | 194 |
| Ia-18 | Cl | Cl | - | - | H | $-CH_2-CH_2-CH_2-CH_2-$ | | > 220 |
| Ia-19 | $CF_3$ | H | - | - | H | $-CH_2-CH_2-CH_2-CH_2-$ | | 180 |
| Ia-20 | Cl | Cl | - | - | $CH_3$ | H | 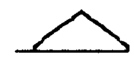 | 165 |
| Ia-21 | Cl | Cl | - | - | $C_2H_5$ | H | $CH_3$ | 178 |
| Ia-22 | Cl | Cl | - | - | $CH_3$ | H | 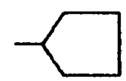 | 188 |
| Ia-23 | Cl | Cl | - | - | $CH_3$ | H | $i-C_3H_7$ | > 220 |
| Ia-24 | Cl | Cl | - | - | $CH_3$ | H | $i-C_4H_9$ | 150 |
| Ia-25 | Cl | Cl | - | - | $CH_3$ | H | $C_2H_5$ | 140 |
| Ia-26 | Br | H | - | - | $CH_3$ | H | $CH_3$ | 198 |
| Ia-27 | H | H | - | - | H | H | $-CH_2-$ 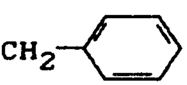 | 164 |

<u>Tabelle 3</u> (Fortsetzung)

| Bsp.Nr. | X | Y | Z | n | M | L | A | Schmelzpunkt/°C |
|---|---|---|---|---|---|---|---|---|
| Ia-28 | H | H | - | - | H | H | 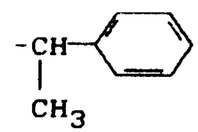 | 161 |
| Ia-29 | Cl | Cl | - | - | 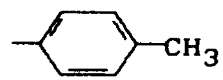 | H | $CH_3$ | 155-156 |
| Ia-30 | Cl | Cl | - | - | 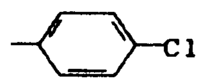 | H | $CH_3$ | 145-150 |
| Ia-31 | Cl | Cl | - | - | 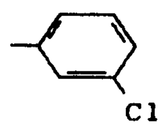 | H | $CH_3$ | 175-180 |
| Ia-32 | Cl | Cl | - | - | 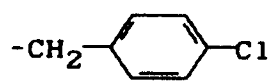 | H | $CH_3$ | 195-200 |
| Ia-33 | Cl | Cl | - | - | 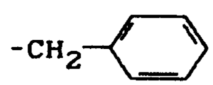 | H | $CH_3$ | 205-208 |

<u>Tabelle 3</u> (Fortsetzung)

| Bsp.Nr. | X | Y | Z | n | M | L | A | Schmelzpunkt/°C |
|---|---|---|---|---|---|---|---|---|
| Ia-34 | Cl | Cl | - | - | $-CH_2$ 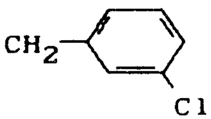 | H | $CH_3$ | 225-230 |
| Ia-35 | Cl | Cl | - | - | $-CH_2$ 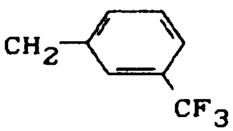 | H | $CH_3$ | 185-190 |
| Ia-36 | Cl | Cl | - | - | $-CH_2$ 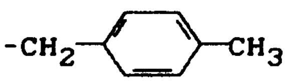 | H | $CH_3$ | 210-215 |
| Ia-37 | Cl | Cl | - | - | 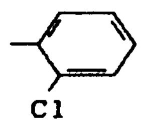 | H | $CH_3$ | 180-185 |
| Ia-38 | Cl | Cl | - | - | 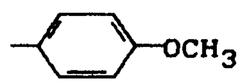 | H | $CH_3$ | 183-184 |
| Ia-39 | Cl | Cl | - | - | H | H | 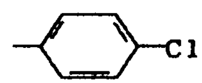 • | 260-262 |
| Ia-40 | Cl | Cl | - | - | 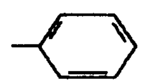 | H | $CH_3$ | 202-204 |

Tabelle 3 (Fortsetzung)

| Bsp.Nr. | X | Y | Z | n | M | L | A | Schmelzpunkt/°C |
|---|---|---|---|---|---|---|---|---|
| Ia-41 | Cl | H | - | - | H | H | 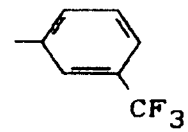 | Harz |
| Ia-42 | $CF_3$ | H | - | - | 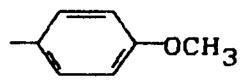 | H | $CH_3$ | 135-7 |
| Ia-43 | $CF_3$ | H | - | - | 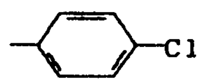 | H | $CH_3$ | 168 |
| Ia-44 | $CF_3$ | H | - | - | 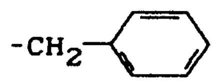 | H | $CH_3$ | 142 |
| Ia-45 | $CF_3$ | H | - | - | 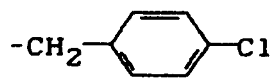 | H | $CH_3$ | 160-2 |
| Ia-46 | $CF_3$ | H | - | - | 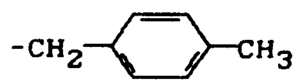 | H | $CH_3$ | 170 |
| Ia-47 | $CF_3$ | H | - | - | 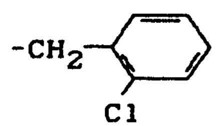 | H | $CH_3$ | 50-3 |

**Tabelle 3** (Fortsetzung)

| Bsp.Nr. | X | Y | Z | n | M | L | A | Schmelzpunkt/°C |
|---|---|---|---|---|---|---|---|---|
| Ia-48 | $CF_3$ | H | - | - | 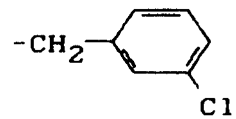 | H | $CH_3$ | 122 |
| Ia-49 | $CF_3$ | H | - | - | 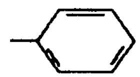 | H | $CH_3$ | 140-2 |
| Ia-50 | $CF_3$ | H | - | - | 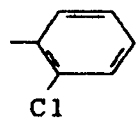 | H | $CH_3$ | 130 |
| Ia-51 | $CF_3$ | H | - | - | 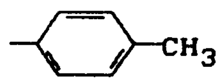 | H | $CH_3$ | 152 |
| Ia-52 | $CF_3$ | H | - | - | 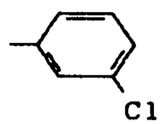 | H | $CH_3$ | 162-5 |
| Ia-53 | $CF_3$ | H | - | - | 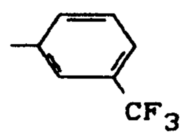 | H | $CH_3$ | 124-5 |

Tabelle 3 (Fortsetzung)

| Bsp.Nr. | X | Y | Z | n | M | L | A | Schmelzpunkt/° C |
|---|---|---|---|---|---|---|---|---|
| Ia-54 | $CF_3$ | H | - | - | H | H | 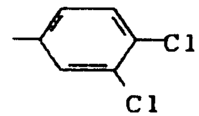 | 236 |
| Ia-55 | $CF_3$ | H | - | - | H | H | $-CH_2-CH_2-$ 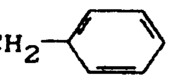 | 114-116 |
| Ia-56 | $CF_3$ | H | - | - | H | H | $-CH_2-$ 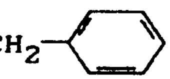 | 139-141 |
| Ia-57 | $CF_3$ | H | - | - | $CH_3$ | H | $-CH_2-CH_2-$ 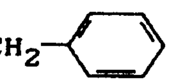 | 148-150 |
| Ia-58 | $CF_3$ | H | - | - | $CH_3$ | H | $-CH_2-$ 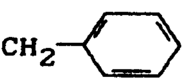 | 116-118 |
| Ia-59 | $CF_3$ | H | - | - | H | $-(CH_2)_2-S-CH_2-$ | | 188-190 |

**Tabelle 3** (Fortsetzung)

| Bsp.Nr. | X | Y | Z | n | M | L | A | Schmelzpunkt/° C |
|---|---|---|---|---|---|---|---|---|
| Ia-60 | $CF_3$ | H | - | - | H | $-(CH_2)_3-$ | | 184-186 |
| Ia-61 | $CF_3$ | H | - | - | H | H | $-CH_2-C(CH_3)_3$ | 178-180 |
| Ia-62 | $CF_3$ | H | - | - | $CH_3$ | H | $-CH_2-C(CH_3)_3$ | 173-175 |
| Ia-63 | $CF_3$ | H | - | - | H | 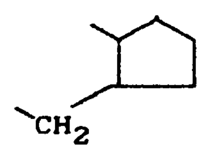 | | 232-234 |

Beispiel 5

Synthese der Zwischenprodukte (III)

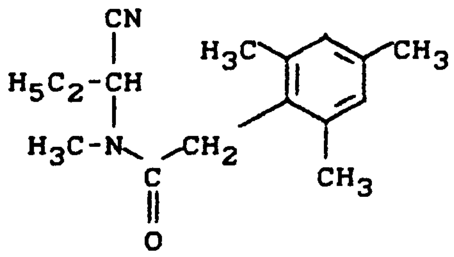

(III)

MW 258.36

N-(α-Cyanopropyl)-N-methyl-2,4,6-trimethylphenylessigsäureamid

9,82 g (0,1 Mol) N-(α-Gyanopropyl)-methylamin werden mit 10,12 g Triethylamin in 120 ml Tetrahydrofuran gelöst und auf 0 ° C gekühlt. Sodann werden 19,28 g (0,098 Mol) 2,4,6-Trimethylphenylessigsäurechlorid über eine Stunde hinweg bei 0 ° C zugetropft. Nach Beendigung des Vorganges trägt man die Lösung in ein Gemisch aus 500 ml Eisessig und 50 ml 1 normaler HCl-Lösung ein, filtriert den dabei anfallenden Niederschlag ab und trocknet.

Man erhält 22,9 g (91 % der Theorie) N-(α-Cyanopropyl)-N-methyl-2,4,6-trimethylphenylessigsäureamid.

Beispiel 6

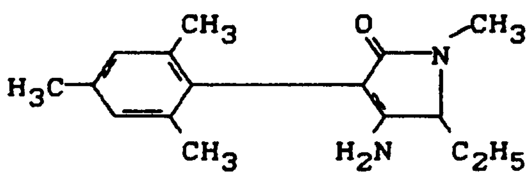

(Ia-41)

MW 258.4

4-Amino-5-ethyl-1-methyl-3-(2,4,6-trimethylphenyl)-pyrrolin-2-on

Verfahren aβ

1,2 g (0,12 Mol) Natriumhydrid werden in 60 ml absolutem Toluol gelöst und 22,5 g N-(α-Cyanopropyl)-N-methyl-2,4,6-trimethylphenylessigsäureamid, gelöst in 100 ml absolutem Toluol, unter Eisbadkühlung zugetropft. Anschließend läßt man solange rühren, bis kein Wasserstoff mehr entweicht. Danach engt man die Reaktionslösung im Vakuum ein, nimmt den Rückstand in Wasser auf, säuert bei 0-20 ° C mit konzentrierter HCl-Lösung an und filtriert den dabei anfallenden Niederschlag ab. Dieser wird bei 70 ° C über Phosphorpentoxid im Vakuum getrocknet, nachdem er gegebenenfalls zuvor mit einem Gemisch aus Chloroform, Methyl-tertiärbutylether und n-Hexan aus gekocht wurde.

Man erhält 17,4 g (77,4 % der Theorie) 4-Amino-5-ethyl-1-methyl-3-(2,4,6-trimethylphenyl)-pyrrolin-2-on mit dem Schmelzpunkt von 192 ° C.

Beispiel 7

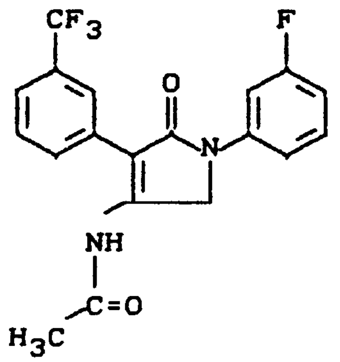

(Ib-1)

MW 378

4-Acetylamino-1-(3-fluorphenyl)-3-(3-trifluormethylphenyl)-pyrrolin-2-on

Verfahren bα

2,0 g (5,95 mMol) 4-Amino-1-(3-fluorphenyl)-3-(3-trifluormethylphenyl)-pyrrolin-2-on (Ia-1) werden mit 40 ml Essigsäurechlorid 4 Stunden zum Rückflußsieden erhitzt. Die dabei resultierende Suspension wird unter Eisbadkühlung in 250 ml Eiswasser eingetragen, dreimal mit je 100 ml Methylenchlorid extrahiert, die gesammelten organischen Phasen mit gesättigter Ammoniumchlorid-Lösung gewaschen, mit Magnesiumsulfat getrocknet und schließlich das Methylenchlorid im Vakuum wieder entfernt.

Der dabei anfallende hellgraue Feststoff wird mit Methyl-tertiär-butylether verrührt, die Aufschlämmung abfiltriert und der Feststoff getrocknet.

Man erhält 1,3 g (57,8 % der Theorie) 4-Acetylamino-1-(3-fluorphenyl)-3-(3-trifluormethylphenyl)-pyrrolin-2-on mit einem Schmelzpunkt von 115 °C.

Analoge Umsetzungen werden nach dieser allgemeinen Vorschrift durchgeführt. Die Ausbeuten differieren zwischen 35 und 90 %. In Einzelfällen wurde über Kieselgel (Laufmittel Toluol:Aceton (3:1) chromatographiert.

In analoger Weise zu Beispiel 7 und unter Berücksichtigung der Angaben in der Beschreibung zum erfindungsgemäßen Verfahren (bα) erhält man die nachfolgend in Tabelle 4 aufgeführten Endprodukte der Formel (Ib), in denen B für Acetyl steht.

<u>Tabelle 4</u>

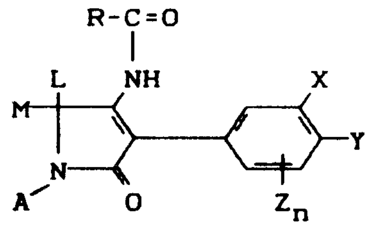

(Ib)

| Bsp.Nr. | X | Y | Z | n | L | M | A | R | Schmelzpunkt/$^{0}$C |
|---|---|---|---|---|---|---|---|---|---|
| Ib-2 | $CF_3$ | H | - | - | $CH_3$ | H | 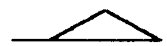 | $CH_3$ | 80 |
| Ib-3 | $CF_3$ | H | - | - | $-CH_2$ 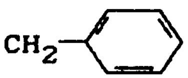 | H | H | $CH_3$ | 182-183 |
| Ib-4 | 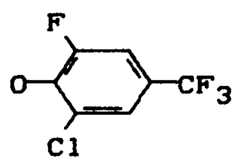 | H | - | - | H | H | 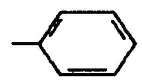 | $CH_3$ | 162-163 |
| Ib-5 | $CF_3$ | H | - | - | $CH_3$ | H | $CH_3$ | $CH_3$ | Harz |
| Ib-6 | $CF_3$ | H | - | - | H | H | 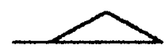 | $CH_3$ | 145 |
| Ib-7 | $CF_3$ | H | - | - | $CH_3$ | H | $C_2H_5$ | $CH_3$ | 70 |

Tabelle 4 (Fortsetzung)

| Bsp.Nr. | X | Y | Z | n | L | M | A | R | Schmelzpunkt/° C |
|---|---|---|---|---|---|---|---|---|---|
| Ib-8 | $CF_3$ | H | - | - | $CH_3$ | H | 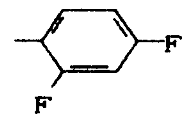 | $CH_3$ | 96 |
| Ib-9 | $CF_3$ | H | - | - | H | H | i-$C_3H_7$ | $CH_3$ | 143 |
| Ib-10 | Cl | Cl | - | - | $CH_3$ | H | $CH_3$ | $CH_3$ | 183 |
| Ib-11 | H | H | - | - | H | H | 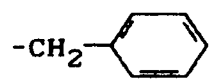 | $CH_3$ | 219 |
| Ib-12 | H | H | - | - | H | H | 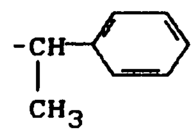 | $CH_3$ | 78 |
| Ib-13 | $CF_3$ | H | - | - | H | H | 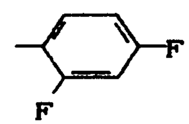 | $CH_3$ | 236-237 |

<u>Tabelle 4</u> (Fortsetzung)

| Bsp.Nr. | X | Y | Z | n | M | L | A | R | Schmelzpunkt/°C |
|---|---|---|---|---|---|---|---|---|---|
| Ib-14 | $CF_3$ | H | - | - | H | H | 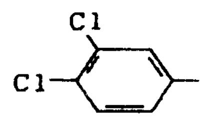 | $CH_3$ | 202 |
| Ib-15 | Cl | H | - | - | H | H | 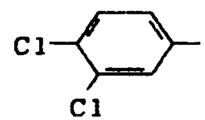 | $CH_3$ | 234 |
| Ib-16 | $CF_3$ | H | - | - | H | $-(CH_2)_4-$ | | $CH_3$ | 165 |
| Ib-17 | Cl | Cl | - | - | H | $CH_3$ | $i\text{-}C_4H_9-$ | $CH_3$ | 88-98 |
| Ib-18 | Cl | Cl | - | - | H | $CH_3$ | $C_2H_5-$ | $CH_3$ | 143-145 |
| Ib-19 | Cl | Cl | - | - | H | $CH_3$ | 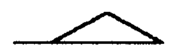 | $CH_3$ | 184-187 |
| Ib-20 | Cl | Cl | - | - | H | $C_2H_5$ | $CH_3$ | $CH_3$ | 168-169 |
| Ib-21 | Cl | Cl | - | - | H | $CH_3$ | 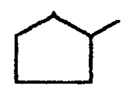 | $CH_3$ | 190-193 |

<u>Tabelle 4</u> (Fortsetzung)

| Bsp.Nr. | X | Y | Z | n | M | L | A | R | Schmelzpunkt/°C |
|---|---|---|---|---|---|---|---|---|---|
| Ib-22 | Cl | Cl | - | - | $CH_3$ | H | i-$C_3H_7$ | $CH_3$ | Harz |
| Ib-23 | Cl | Cl | - | - | H | H | 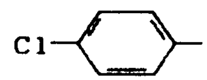 | $CH_3$ | 265 |
| Ib-24 | Cl | H | - | - | H | H | 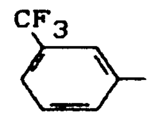 | $CH_3$ | 225-226 |
| Ib-25 | $CF_3$ | H | - | - | 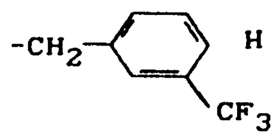 | H | $CH_3$ | $CH_3$ | 72 |
| Ib-26 | Cl | Cl | - | - | H | H | 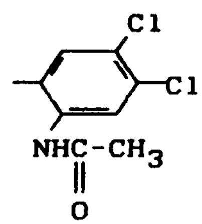 | $CH_3$ | >250 |
| Ib-27 | $CF_3$ | H | - | - | H | H | 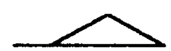 | $C_2H_5$ | 117 |

<u>Tabelle 4</u> (Fortsetzung)

| Bsp.Nr. | X | Y | Z | n | M | L | A | R | Schmelzpunkt/°C |
|---|---|---|---|---|---|---|---|---|---|
| Ib-28 | $CF_3$ | H | - | - | H | H | 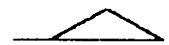 | $C(CH_3)_3$ | 132 |
| Ib-29 | $CF_3$ | H | - | - | H | H | 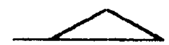 | $CH_2$-$C(CH_3)_3$ | 153 |
| Ib-30 | $CF_3$ | H | - | - | $CH_2$ 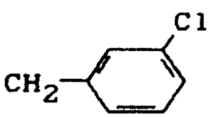 | H | $CH_3$ | $CH_3$ | 54-57 |
| Ib-31 | $CF_3$ | H | - | - | $CH_2$ 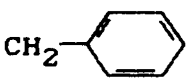 | H | $CH_3$ | $CH_3$ | 115-117 |
| Ib-32 | $CF_3$ | H | - | - | $CH_2$ 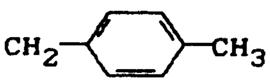 | H | $CH_3$ | $CH_3$ | 145-147 |
| Ib-33 | $CF_3$ | H | - | - | 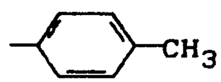 | H | $CH_3$ | $CH_3$ | 75- 77 |

<u>Tabelle 4</u> (Fortsetzung)

| Bsp.Nr. | X | Y | Z | n | M | L | A | R | Schmelzpunkt/°C |
|---|---|---|---|---|---|---|---|---|---|
| Ib-34 | $CF_3$ | H | - | - | 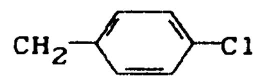 | H | $CH_3$ | $CH_3$ | 155-157 |
| Ib-35 | $CF_3$ | H | - | - | H | H | 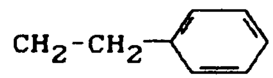 | $CH_3$ | 63- 66 |
| Ib-36 | $CF_3$ | H | - | - | H | H | 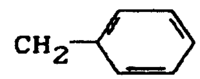 | $CH_3$ | 112-114 |
| Ib-37 | $CF_3$ | H | - | - | $CH_3$ | H | 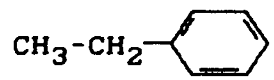 | $CH_3$ | 73- 76 |
| Ib-38 | $CF_3$ | H | - | - | $CH_3$ | H | 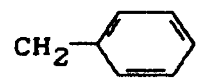 | $CH_3$ | 77- 80 |
| Ib-39 | $CF_3$ | H | - | - | 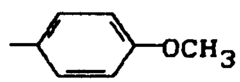 | H | $CH_3$ | $CH_3$ | 134-137 |

**Tabelle 4** (Fortsetzung)

| Bsp.Nr. | X | Y | Z | n | M | L | A | R | Schmelzpunkt/° C |
|---|---|---|---|---|---|---|---|---|---|
| Ib-40 | $CF_3$ | H | - | - | H | $-CH_2-CH_2-S-CH_2-$ | | $CH_3$ | 199-201 |
| Ib-41 | $CF_3$ | H | - | - | H | $-CH_2-CH_2-CH_2-$ | | $CH_3$ | 128-130 |
| Ib-42 | $CF_3$ | H | - | - | H | H | $CH_2-C(CH_3)_3$ | $CH_3$ | 113-115 |
| Ib-43 | $CF_3$ | H | - | - | $CH_3$ | H | $CH_2-C(CH_3)_3$ | $CH_3$ | 159-160 |
| Ib-44 | $CF_3$ | H | - | - | 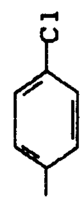 | H | $CH_3$ | $CH_3$ | 93- 95 |

## Anwendungsbeispiele

In den folgenden Anwendungsbeispielen wurde die nachstehend aufgeführte Verbindung als Vergleichssubstanz eingesetzt:

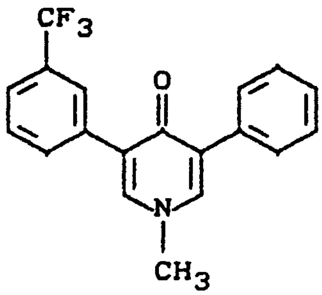

(A)

1-Methyl-3-phenyl-5-(2,α,α-trifluor-m-tolyl)-4-pyridon [Fluridone] bekannt aus R. Wegler, Chemie der Pflanzenschutz und Schädlingsbekämpfungsmittel 5, Herbizide, Axel-Springer-Verlag, Berlin, Heidelberg, New York 1977, Seite 309.

Beispiel A

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

Eine deutliche Überlegenheit in der Wirksamkeit ebenso wie in der Nutzpflanzenselektivität gegenüber dem Stand der Technik zeigen in diesem Test z.B. die Verbindungen gemäß folgender Herstellungsbeispiele Ia-2, Ia-3, Ia-11, Ib-6 und Ib-9.

Beispiel B

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstante Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

Eine deutliche Überlegenheit in der Wirksamkeit ebenso wie in der Nutzpflanzenselektivität gegenüber dem Stand der Technik zeigen in diesem Test z.B. die Verbindungen gemäß folgendem Herstellungsbeispiel: Ib-9.

**Patentansprüche**

1. 4-(substituierte) Amino-3-arylpyrrolinon-Derivate der allgemeinen Formel (I)

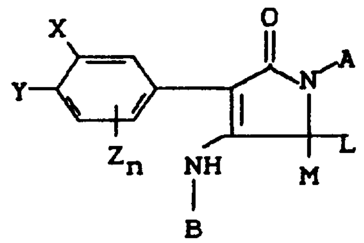

(I),

dadurch gekennzeichnet, daß

X und Y unabhängig voneinander für Wasserstoff, Halogen, Alkyl, Alkoxy, Alkylthio, Halogenalkoxy, Halogenalkyl, oder jeweils für einen unsubstituierten oder substituierten Rest der Reihe Aryl, Aryloxy oder Arylthio stehen,

Z für Halogen, Alkyl oder Alkoxy steht,

n für eine Zahl 0, 1, 2 oder 3 steht,

A für einen jeweils unsubstituierten oder jeweils durch Halogen substituierten Rest der Reihe Alkyl, Alkenyl, Alkinyl, Alkoxyalkyl, Halogenalkyl, Polyalkoxyalkyl oder Alkylthioalkyl, gegebenenfalls durch Heteroatome unterbrochenes Cycloalkyl, oder für unsubstituiertes oder durch Halogen, Alkyl, Halogenalkyl, Alkoxy und/oder Nitro substituiertes Arylalkyl oder unsubstituiertes oder substituiertes Aryl steht, wobei als Substituenten Halogen, Alkyl, Alkoxy, Halogenalkyl, Halogenalkoxy, jeweils unsubstituiertes oder durch Halogen, Alkyl, Alkoxy, Halogenalkyl und/oder Halogenalkoxy substituiertes Phenoxy oder Phenylthio in Frage kommen,

B für Wasserstoff, für die Gruppe

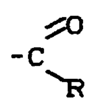

oder für die Gruppe

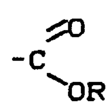

steht,

L für Wasserstoff, oder für einen gegebenenfalls durch Halogen substituierten Rest der Reihe Alkyl, Alkenyl, Alkinyl, Polyalkoxyalkyl, Alkoxyalkyl, Alkylthioalkyl, gegebenenfalls durch Heteroatome unterbrochenes Cycloalkyl oder für einen gegebenenfalls durch Halogen, Alkyl, Halogenalkyl, Alkoxy oder Nitro substituierten Rest der Reihe Aryl, Arylalkyl oder Hetaryl steht,

M für Wasserstoff, Alkyl oder Alkoxyalkyl steht,

oder

A und L oder L und M gemeinsam mit den Atomen, an die sie gebunden sind einen Cyclus bilden und

R für einen gegebenenfalls durch Halogen substituierten Rest der Reihe Alkyl, Alkenyl, Alkinyl, Alkoxyalkyl, Polyalkoxyalkyl, Alkylthioalkyl, gegebenenfalls durch Heteroatome unterbrochenes Cycloalkyl oder für ein gegebenenfalls durch Halogen, Alkyl, Halogenalkyl, Alkoxy oder Nitro substituiertes Aryl steht,

sowie die enantiomeren Formen von Verbindungen der Formel (I), ausgenommen die Verbindungen 4-Amino-1-isopropyl-3-(3-trifluormethylphenyl)-3-pyrrolin-2-on und 4-Amino-1-cyclopropyl-3-(3-trifluormethylphenyl)-3-pyrrolin-2-on.

**2.** 4-(substituierte) Amino-3-arylpyrrolinon-Derivate der allgemeinen Formel (I), gemäß Anspruch 1, dadurch gekennzeichnet, daß

X und Y unabhängig voneinander für Wasserstoff, Fluor, Chlor, Brom, Iod, $C_1$-$C_6$-Alkyl, $C_1$-$C_6$-Alkoxy, $C_1$-$C_6$-Alkylthio, $C_1$-$C_6$-Halogenalkoxy, Halogen-$C_1$-$C_4$-alkyl, oder jeweils für einen unsubstituierten oder einfach bis fünffach, gleich oder verschieden substituierten Rest der Reihe Phenyl, Phenoxy oder Phenylthio stehen, wobei als Phenylsubstituenten ausgewählt sind: Fluor, Chlor, Brom, Iod, $C_1$-$C_4$-Alkyl, $C_1$-$C_4$-Alkoxy und Halogen-$C_1$-$C_4$-alkyl,

Z für Fluor, Chlor, Brom, Iod, $C_1$-$C_6$-Alkyl oder $C_1$-$C_6$-Alkoxy steht,

n für eine Zahl 0, 1, 2 oder 3 steht,

A für einen jeweils unsubstituierten oder jeweils durch Halogen substituierten Rest der Reihe $C_1$-$C_{12}$-Alkyl, $C_3$-$C_8$-Alkenyl, $C_3$-$C_8$-Alkinyl, Halogen-$C_1$-$C_8$-alkyl, $C_1$-$C_{10}$-Alkoxy-$C_2$-$C_8$-alkyl, $C_1$-$C_8$-Polyalkoxy-$C_1$-$C_8$-alkyl, $C_1$-$C_{10}$-Alkylthio-$C_1$-$C_8$-alkyl oder für Cycloalkyl mit 3 bis 8 Ringatomen steht, das durch Sauerstoff, Stickstoff und/oder Schwefel unterbrochen sein kann, oder A für einen jeweils unsubstituierten oder im Phenylteil einfach bis fünffach, gleich oder verschieden substituierten Rest der Reihe Phenyl-$C_1$-$C_6$-alkyl, Phenyl oder Naphthyl steht, wobei als Substituenten jeweils susgewählt sind: Fluor, Chlor, Brom, Iod, $C_1$-$C_6$-Alkyl, $C_1$-$C_6$-Alkoxy, Halogen-$C_1$-$C_6$-alkyl, Halogen-$C_1$-$C_6$-alkoxy oder jeweils unsubstituiertes oder einfach bis fünffach, gleich oder verschieden durch Fluor, Chlor, Brom, Iod, $C_1$-$C_4$-Alkyl, $C_1$-$C_4$-Alkoxy, Halogen-$C_1$-$C_4$-alkyl und Halogen-$C_1$-$C_4$-alkoxy substituiertes Phenoxy oder Phenylthio,

B für Wasserstoff, für die Gruppe

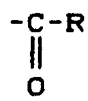

oder für die Gruppe

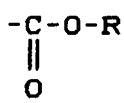

steht,

L für Wasserstoff, oder für einen gegebenenfalls durch Halogen substituierten geradkettigen oder verzweigten Rest der Reihe $C_1$-$C_{12}$-Alkyl, $C_3$-$C_8$-Alkenyl, $C_3$-$C_8$-Alkinyl, $C_1$-$C_{10}$-Alkoxy-$C_2$-$C_8$-alkyl, $C_1$-$C_8$-Polyalkoxy-$C_2$-$C_8$-alkyl, $C_1$-$C_{10}$-Alkylthio- $C_2$-$C_8$-alkyl, Cycloalkyl mit 3-8 Ringatomen, das durch Sauerstoff und/oder Schwefel unterbrochen sein kann, oder für einen gegebenenfalls durch Halogen, $C_1$-$C_6$-Alkyl-$C_1$-$C_6$-halogenalkyl, $C_1$-$C_6$-Alkoxy oder Nitro substituierten Rest der Reihe Aryl, Hetaryl oder Aryl-$C_1$-$C_6$-alkyl steht,

M für Wasserstoff oder für einen geradkettigen oder verzweigten Rest der Reihe $C_1$-$C_{12}$-Alkyl, $C_1$-$C_8$-Alkoxyalkyl steht,

oder worin

A und L oder L und M gemeinsam mit den Atomen, an die sie gebunden sind, einen 3 bis 8-gliedrigen Ring bilden, der gesättigt oder ungesättigt sein kann und der substituiert sein kann und/oder durch Sauerstoff/Schwefel unterbrochen sein kann,

R für einen gegebenenfalls durch Halogen substituierten geradkettigen oder verzweigten Rest der Reihe $C_1$-$C_{12}$-Alkyl, $C_3$-$C_8$-Alkenyl, $C_3$-$C_8$-Alkinyl, $C_1$-$C_{10}$-Alkoxy-$C_2$-$C_8$-alkyl, $C_1$-$C_8$-Polyalkoxy-$C_2$-$C_8$-alkyl, $C_1$-$C_{10}$-Alkylthio-$C_2$-$C_8$-alkyl, Cycloalkyl mit 3-8 Ringatomen, das durch Sauerstoff und/oder Schwefel unterbrochen sein kann, oder für gegebenenfalls durch Halogen, $C_1$-$C_6$-Alkyl-$C_1$-$C_6$-halogen-alkyl, $C_1$-$C_6$-Alkoxy oder Nitro substituiertes Aryl, steht,

sowie die enantiomeren Formen von Verbindungen der Formel (I) ausgenommen die Verbindungen: 4-Amino-1-cyclopropyl-3-(3-trifluormethylphenyl)-3-pyrrolin-2-on und 4-Amino-1-isopropyl-3-(3-trifluormethylphenyl)-3-pyrrolin-2-on.

3. 4-(substituierte)Amino-3-arylpyrrolinon-Derivate der allgemeinen Formel (I) gemäß Anspruch 1, dadurch gekennzeichnet, daß

X für Wasserstoff, Fluor, Chlor, Brom, Methyl, Ethyl, n-oder iso-Propyl, Methoxy, Ethoxy, n-oder i-Propoxy, Halogenmethyl mit 1, 2 oder 3 Fluor- und/oder Chloratomen, Halogenethyl mit 1 bis 5, insbesondere 1 bis 3, Fluor- und/oder Chloratomen, $C_1$-$C_3$-Halogenalkoxy, $C_1$-$C_3$-Alkylthio, jeweils unsubstituiertes oder einfach bis fünffach, insbesondere einfach bis dreifach, gleich oder verschieden substituiertes Phenyl, Phenoxy oder Phenylthio steht, wobei als Phenylsubstituenten ausgewählt sind: Fluor, Chlor, Brom, Methyl, Ethyl, n- oder iso-Propyl, Methoxy, Ethoxy, n- oder iso-Propoxy und Trifluormethyl,

Y für Wasserstoff, Fluor, Chlor, Brom, Methyl, Ethyl, Methoxy, Ethoxy, Trifluormethoxy oder Trifluormethyl steht,

Z für Fluor oder Chlor steht,

n für 0, 1, 2 oder 3 steht und

A für Methyl, Ethyl, für einen jeweils geradkettigen oder verzweigten Rest der Reihe Propyl, Butyl, Pentyl, Hexyl, Heptyl, Octyl, Nonyl, Decyl, Halogen-$C_1$-$C_6$-alkyl, $C_3$-$C_6$-Alkenyl, $C_3$-$C_6$-Alkinyl, $C_1$-$C_4$-Alkoxy-$C_1$-$C_4$-alkyl, $C_1$-$C_4$-Alkylthio-$C_1$-$C_4$-alkyl steht, oder für Cycloalkyl mit 3 bis 6 Ringatomen, das durch Sauerstoff, Stickstoff oder Schwefel unterbrochen sein kann steht, oder A für einen jeweils unsubstituierten oder einfach bis fünffach, insbesondere einfach bis dreifach, gleich oder verschieden substituierten Rest der Reihe Phenyl, Benzyl oder Phenethyl steht, wobei als Substituenten jeweils ausgewählt sind: Fluor, Chlor, Methyl, Ethyl, n-oder iso-Propyl, Methoxy, Ethoxy, Trifluormethyl, Trifluormethoxy oder jeweils unsubstituiertes oder einfach bis dreifach, gleich oder verschieden, durch Fluor, Chlor, Methyl, Methoxy, Trifluormethyl und Trifluormethoxy substituiertes Phenoxy oder Phenylthio,

B für Wasserstoff, für die Gruppe

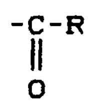

oder für die Gruppe

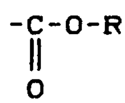

steht,

L für Wasserstoff, oder für einen gegebenenfalls durch Halogen substituierten geradkettigen oder verzweigten Rest der Reihe $C_1$-$C_{10}$-Alkyl, $C_3$-$C_6$-Alkenyl, $C_3$-$C_6$-Alkinyl, $C_1$-$C_8$-Alkoxy-$C_2$-$C_6$-alkyl, $C_1$-$C_6$-Polyalkoxy-$C_2$-$C_6$-alkyl, $C_1$-$C_8$-Alkylthio-$C_2$-$C_6$-alkyl, Cycloalkyl mit 3 bis 7 Ringatomen, das durch 1 bis 2 Sauerstoff- und/oder Schwefelatome unterbrochen sein kann oder gegebenenfalls durch Halogen-, $C_1$-$C_4$-Alkyl, $C_1$-$C_4$-Halogenalkyl, $C_1$-$C_4$-Alkoxy oder Nitro substituiertes Aryl, steht,

M für Wasserstoff, oder für einen geradkettigen oder verzweigten Rest der Reihe $C_1$-$C_{10}$-Alkyl, $C_1$-$C_6$-Alkoxyalkyl steht,

oder worin

A und L oder L und M gemeinsam mit den Atomen an die sie gebunden sind, einen 3 bis 7-gliedrigen Ring bilden, der gesättigt oder ungesättigt sein kann, der durch $C_1$-$C_6$-Alkyl, $C_1$-$C_4$-Alkoxy, $C_1$-$C_2$-Halogenalkyl oder Phenyl sustituiert sein kann und/oder durch Sauerstoff/Schwefel unterbrochen sein kann,

R für einen gegebenenfalls durch Halogen substituierten geradkettigen oder verzweigten Rest der Reihe $C_1$-$C_{10}$-Alkyl, $C_3$-$C_6$-Alkenyl, $C_3$-$C_6$-Alkinyl, $C_1$-$C_8$-Alkoxy-$C_2$-$C_6$-alkyl, $C_1$-$C_6$-Polyalkoxy-$C_2$-$C_6$-alkyl, $C_1$-$C_8$-Alkylthio-$C_2$-$C_6$-alkyl, Cycloalkyl mit 3 bis 7 Ringatomen, das durch 1 bis 2 Sauerstoff- und/oder Schwefelatome unterbrochen sein kann oder für gegebenenfalls durch Halogen-, $C_1$-$C_4$-Alkyl-, $C_1$-$C_4$-Halogenalkyl, $C_1$-$C_4$-Alkoxy-, Nitro substituiertes Aryl, steht,

sowie die enantiomeren Verbindungen der Formel (I) ausgenommen die Verbindungen:

4-Amino-1-cyclopropyl-3-(3-trifluormethylphenyl)-3-pyrrolin-2-on und 4-Amino-1-isopropyl-3-(3-trifluormethylphenyl)-3-pyrrolin-2-on.

**4.** 4-(substituierte) Amino-3-arylpyrrolinon-Derivate der allgemeinen Formel (I) gemäß Anspruch 1, dadurch gekennzeichnet, daß

X für Wasserstoff, Fluor, Chlor, Brom, Methyl, Ethyl, Methoxy, Ethoxy, n- oder i-Propoxy, Trifluormethyl, Trifluormethoxy oder für jeweils unsubstituiertes oder einfach bis dreifach, gleich oder verschieden substituiertes Phenyl oder Phenoxy steht, wobei als Substituenten ausgewählt sind: Fluor, Chlor, Methyl und Trifluormethyl,

Y für Wasserstoff, Fluor, Chlor, Brom, Methyl, Methoxy, Trifluormethoxy oder Trifluormethyl steht,

n für 0 steht und

A für Methyl, Ethyl, n-Propyl, iso-Propyl, n-, iso-, sec.- oder tert.-Butyl, für einen jeweils geradkettigen oder verzweigten Rest der Reihe Pentyl, Hexyl, Heptyl, Octyl, Halogen-$C_1$-$C_3$-alkyl, Allyl, Propargyl, Methoxymethyl, Methoxyethyl, Ethoxymethyl, Ethoxyethyl, Cyclopropyl, Cyclopentyl, Cyclohexyl, oder für jeweils unsubstituiertes oder einfach bis dreifach, gleich oder verschieden durch Fluor, Chlor, Trifluormethyl, Phenoxy und 4-Trifluormethyl-phenoxy substituiertes Phenyl oder Benzyl steht,

B für Wasserstoff, für die Gruppe -CO-R oder für die Gruppe

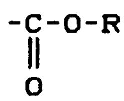

steht,

L für Wasserstoff, oder für einen gegebenenfalls durch Halogen substituierten geradkettigen oder verzweigten Rest der Reihe $C_1$-$C_8$-Alkyl, $C_3$-$C_4$-Alkenyl, $C_3$-$C_4$-Alkinyl, $C_1$-$C_6$-Alkoxy-$C_2$-$C_4$-alkyl, $C_1$-$C_4$-Polyalkoxy-$C_2$-$C_4$-alkyl, $C_1$-$C_6$-Alkylthio-$C_2$-$C_4$-alkyl, Cycloalkyl mit 3 bis 6 Ringatomen, das durch 1 bis 2 Sauerstoff- und/oder Schwefelatome unterbrochen sein kann oder gegebenenfalls durch Fluor, Chlor, Brom, Methyl, Ethyl, Propyl, iso-Propyl, Methoxy, Ethoxy, Trifluormethyl oder Nitro substituiertes Aryl steht,

M für Wasserstoff, oder für einen geradkettigen oder verzweigten Rest der Reihe $C_1$-$C_8$-Alkyl, $C_1$-$C_4$-Alkoxyalkyl steht,

oder worin

A und L oder L und M gemeinsam mit den Atomen an die sie gebunden sind, einen 3 bis 6-gliedrigen Ring bilden, der gesättigt oder einfach ungesättigt sein kann, der durch $C_1$-$C_4$-Alkyl, $C_1$-$C_2$-Alkoxy, durch Trifluormethyl oder Phenyl substituiert sein kann und/oder durch Sauerstoff/Schwefel unterbrochen sein kann,

R für einen gegebenenfalls durch Halogen substituierten geradkettigen oder verzweigten Rest der Reihe $C_1$-$C_8$-Alkyl, $C_3$-$C_4$-Alkenyl, $C_3$-$C_4$-Alkinyl, $C_1$-$C_6$-Alkoxy-$C_2$-$C_4$-alkyl, $C_1$-$C_4$-Polyalkoxy-$C_2$-$C_4$-alkyl, $C_1$-$C_6$-Alkylthio-$C_2$-$C_4$-alkyl, Cycloalkyl mit 3 bis 6 Ringatomen, das durch 1 bis 2 Sauerstoff- und/oder Schwefelatome unterbrochen sein kann, oder für gegebenenfalls durch Fluor, Chlor, Brom, Methyl, Ethyl, Propyl, iso-Propyl, Methoxy, Ethoxy, Trifluormethyl oder Nitro substituiertes Aryl steht, sowie die enantiomeren Verbindungen der Formel (I) ausgenommen die Verbindungen: 4-Amino-1-cyclopropyl-3-(3-trifluormethylphenyl)-3-pyrrolin-2-on und 4-Amino-1-isopropyl-3-(3-trifluormethylphenyl)-3-pyrrolin-2-on.

**5.** Verfahren zur Herstellung von 4-(substituierten) Amino-3-aryl-pyrrolinon-Derivate der allgemeinen Formel (I)

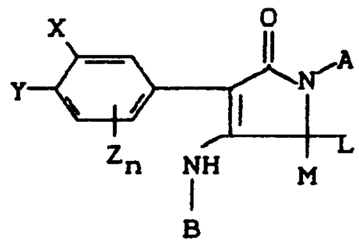

(I)

in welcher

X und Y unabhängig voneinander für Wasserstoff, Halogen, Alkyl, Alkoxy, Alkylthio, Halogenalkoxy, Halogenalkyl, oder jeweils für einen unsubstituierten oder substituierten Rest der Reihe Aryl, Aryloxy oder Arylthio stehen,

Z für Halogen, Alkyl oder Alkoxy steht,

n für eine Zahl O, 1, 2 oder 3 steht,

A für einen jeweils unsubstituierten oder jeweils durch Halogen substituierten Rest der Reihe Alkyl, Alkenyl, Alkinyl, Alkoxyalkyl, Halogenalkyl, Polyalkoxyalkyl oder Alkylthioalkyl, gegebenenfalls durch Heteroatome unterbrochenes Cycloalkyl oder für unsubstituiertes oder durch Halogen, Alkyl, Halogenalkyl, Alkoxy und/oder Nitro substituiertes Arylalkyl oder unsubstituiertes oder substituiertes Aryl steht, wobei als Substituenten Halogen, Alkyl, Alkoxy, Halogenalkyl, Halogenalkoxy, jeweils unsubstituiertes oder durch Halogen, Alkyl, Alkoxy, Halogenalkyl und/oder Halogenalkoxy substituiertes Phenoxy oder Phenylthio in Frage kommen,

B für Wasserstoff, für die Gruppe

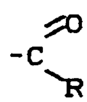

oder für die Gruppe

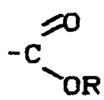

steht,

L für Wasserstoff, oder für einen gegebenenfalls durch Halogen substituierten Rest der Reihe Alkyl, Alkenyl, Alkinyl, Polyalkoxyalkyl, Alkoxyalkyl, Alkylthioalkyl, gegebenenfalls durch Heteroatome unterbrochenes Cycloalkyl oder für einen gegebenenfalls durch Halogen, Alkyl, Halogenalkyl, Alkoxy oder Nitro substituierten Rest der Reihe Aryl, Arylalkyl oder Hetaryl steht,

M für Wasserstoff, Alkyl oder Alkoxyalkyl steht,

oder

A und L oder L und M gemeinsam mit dem den Atomen, an die sie gebunden sind einen Cyclus bilden und

R für einen gegebenenfalls durch Halogen substituierten Rest der Reihe Alkyl, Alkenyl, Alkinyl, Alkoxyalkyl, Polyalkoxyalkyl, Alkylthioalkyl, gegebenenfalls durch Heteroatome unterbrochenes Cycloalkyl oder für ein gegebenenfalls durch Halogen, Alkyl, Halogenalkyl, Alkoxy oder Nitro substituiertes Aryl steht,

sowie die enantiomeren Formen von Verbindungen der Formel (I), ausgenommen die Verbindungen 4-Amino-1-isopropyl-3-(3-trifluormethylphenyl)-3-pyrrolin-2-on und 4-Amino-1-cyclopropyl-3-(3-trifluormethylphenyl)-3-pyrrolin-2-on, dadurch gekennzeichnet, daß man

aα) die Verbindungen der Formel (Ia)

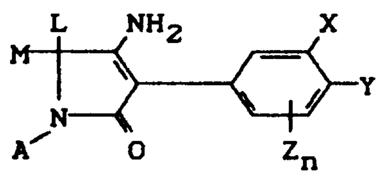

(Ia)

in denen
B für Wasserstoff steht und in welcher die Reste A, L, M, X, Y und $Z_n$ die oben angegebenen Bedeutungen haben,
erhält, wenn man 4-Hydroxypyrrolinone der Formel (II)

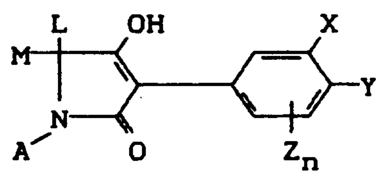

(II)

in denen A, L, M, X, Y, Z und n die oben angegebenen Bedeutungen haben,
mit Ammoniak oder Ammoniaksalzen gegebenenfalls in Gegenwart eines Verdünnungsmittels, gegebenenfalls in Gegenwart eines Katalysators und gegebenenfalls in Gegenwart eines wasserentziehenden Mittels umsetzt,
oder daß man
a$\beta$) Verbindungen der Formel (III)

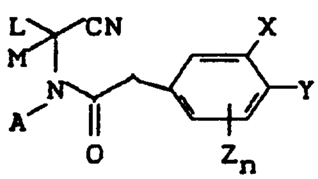

(III)

in denen
A, L, M, X, Y, Z und n die oben angegebenen Bedeutungen haben,
in Gegenwart eines Verdünnungsmittels und in Gegenwart einer Base intramolekular cyclisiert,
oder daß man Verbindungen der Formel (Ib)

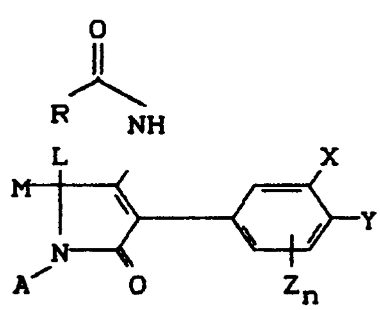

(Ib)

in denen
A, L, M, R, X, Y, Z und n die oben angegebenen Bedeutungen haben,
erhält, wenn man Verbindungen der Formel (Ia)

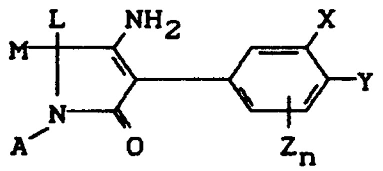

(Ia)

in denen
A, L, M, X, Y, Z und n die oben angegebenen Bedeutungen haben,
bα) mit Säurehalogeniden der allgemeinen Formel (IV)

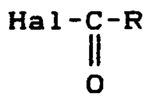

(IV)

in welcher
R die oben angegebene Bedeutung hat und
Hal für Halogen, insbesondere Chlor und Brom steht,
gegebenenfalls in Gegenwart eines Verdünnungsmittels und gegebenenfalls in Gegenwart eines Säurebindemittels umsetzt,
oder
bβ) mit Carbonsäureanhydriden der allgemeinen Formel (V)

R-CO-O-CO-R (V)

in welcher
R die oben angegebene Bedeutung hat,
gegebenenfalls in Gegenwart eines Verdünnungsmittels und gegebenenfalls in Gegenwart eines Säurebindemittels
umsetzt, oder daß man
(c) Verbindungen der Formel (Ic)

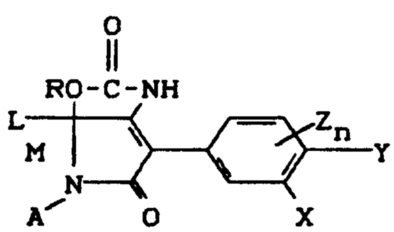

(Ic)

in welcher
A, L, H, X, Y, Z, R und n die oben angegebenen Bedeutungen haben,
erhält, wenn man Verbindungen der Formel (Ia)

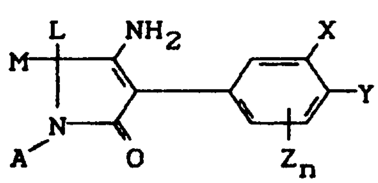

(Ia)

in welcher

A, L, M, X, Y, Z und n die oben angegebenen Bedeutungen haben,
mit Chlorameisensäureester der allgemeinen Formel (VI)

R-O-CO-Cl (VI)

in welcher
R die oben angegebene Bedeutung hat,
gegebenenfals in Gegenwart eines Verdünnungsmittels und gegebenenfalls in Gegenwart eines Säurebindemittels umsetzt.

**6.** Herbizide Mittel, gekennzeichnet durch einen Gehalt an mindestens einem 4-(substituierten)Amino-3-arylpryrrolinon-Derivat der Formel (I) gemäß Anspruch 1 oder 5.

**7.** Verfahren zur Bekämpfung von unerwünschten Pflanzen, dadurch gekennzeichnet, daß man 4-(substituierten)Amino-3-aryl-pyrrolinon-Derivate der Formel (I) gemäß Anspruch 1 oder 5 auf die Pflanzen und/oder ihren Lebensraum einwirken läßt.

**8.** Verwendung von 4-(substituierten)Amino-3-arylpyrrolinon-Derivate der Formel (I) gemäß Anspruch 1 oder 5 zur Bekämpfung von unerwünschten Pflanzen.

**9.** Verfahren zur Herstellung von herbiziden Mitteln, dadurch gekennzeichnet, daß man 4-(substituierte)-Amino-3-arylpyrrolinon-Derivate der Formel (I) gemäß Anspruch 1 oder 5 mit Streckmitteln und/oder oberflächenaktiven Substanzen vermischt.

Europäisches Patentamt

# EUROPÄISCHER RECHERCHENBERICHT

Nummer der Anmeldung

EP 92 12 0660

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| D,A | EP-A-0 262 399 (TAKEDA)<br>6. April 1988<br>* das ganze Dokument *<br>--- | 1-9 | C07D207/38<br>A01N43/36 |
| D,A | EP-A-0 377 893 (BAYER)<br>18. Juli 1990<br>* das ganze Dokument *<br>--- | 1-9 | |
| D,X | EP-A-0 415 185 (BAYER)<br>6. März 1991<br>* Tabelle 2 *<br>* Ansprüche 1-9 *<br>--- | 1-9 | |
| A | US-A-4 643 762 (C. E. WARD)<br>17. Februar 1987<br>* das ganze Dokument *<br>----- | 1-9 | |
| | | | RECHERCHIERTE SACHGEBIETE (Int. Cl.5 ) |
| | | | C07D<br>A01N |

Der vorliegende Recherchenbericht wurde für alle Patentansprüche erstellt

| Recherchenort | Abschlußdatum der Recherche | Prüfer |
|---|---|---|
| DEN HAAG | 02 MAERZ 1993 | Bernd Kissler |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)